# EUROPEAN PATENT APPLICATION

(11) **EP 4 729 300 A2**
(43) Date of publication of application: **22.04.2026**
(21) Application number: 25183993.2
(22) Date of filing: 28.05.2021
(51) Int. Cl.: B33Y 80/00

(54) **IMPLANTS WITH NAVIGATION FIDUCIALS**

(30) Priority: 29.05.2020 US 202063031888 P
(62) Divisional of application: 21742558.6
(71) Applicant: Materialise NV, 3001 Leuven (BE)
(72) Inventor: GEEBELEN, Benjamin, 3001 Leuven (BE)
(74) Representative: Abel & Imray LLP

(57) **Abstract**

A method for providing a bending mold for an off-the-shelf implant, the method comprising obtaining information about a bony anatomy of a patient from one or more medical images; developing a virtual three-dimensional model of the bony anatomy based on the information about the bony anatomy, selecting an off-the-shelf implant, simulating a bending of the off-the-shelf implant using a three-dimensional model of the selected off-the-shelf implant and the virtual three-dimensional model of the bony anatomy to obtain a virtually bent implant having a first, bone-facing, surface and a second surface opposite the first surface, using the virtually bent implant, designing a bending mold mating with the first or the second surface, and based on the design of the bending mold, manufacturing the bending mold using additive manufacturing.

## Description

### Cross-Reference to Related Application(s)

This application claims benefit of and priority to U.S. Provisional Patent Application Serial No. 63/031,888, filed May 29, 2020, herein incorporated by reference in its entirety as if fully set forth below and for all applicable purposes.

### BACKGROUND

### Field of the Disclosure

This application relates to methods and apparatus for manufacturing and using implants with navigation fiducials.

### Description of the Related Technology

Trauma, resection of tumor tissue, congenital anomalies, and other defects can lead to loss of bony tissue or otherwise disrupt bone structures, such as the complex and relatively thin bone structures surrounding and supporting the human eye. For example, such defects in the orbital walls and particularly the orbital floor can cause the eyeball to leave its natural position, potentially leading to blurry vision, an aesthetically unpleasing appearance, and in some cases inability to fully close the eyelids. Such defects present difficult internal bone repair and fixation problems in reconstructive surgery and in trauma surgery.

In some examples, implants can be used for internal repair of defects and fixation of fractures. Typically, an implant may take the form of thin plates. Implants can be manufactured flat or pre-bent to a particular shape, and can be further shaped intra-operatively to fit an individual patient's anatomy. In some examples, an implant may have a patient-specific form, where the shape, circumference, and surface profile are designed to match the individual patient's anatomy. Patient-specific implants generally provide a better fit to the patient anatomy and an easier implantation due to reduced intra-operative shaping.

However, as is typical in orbital implant surgeries, and many other surgeries, the shape and accessibility of the anatomy, such as the eye socket, is such that, upon implantation, certain regions of the implant may be located underneath soft tissue (e.g., and the eyeball), thereby obscuring these regions from view. In an example, if, during surgery, placement of the implant to restore the original position of the eyeball cannot initially be achieved, the surgeon may need to remove and reshape the implant, or possibly even stuff certain areas of the eye socket with graft material, resulting in a potentially lengthy trial-and-error surgery.

It is important to restore the original position of the eyeball as closely as possible, not only for aesthetic reasons, but also to avoid blurred vision. It is therefore important to increase the chance of a close-to-natural initial repositioning of the eyeballs. In view of these and other problems, apparatuses and methods that improve the likelihood of a successful implantation of an implant are described herein.

### SUMMARY

Certain aspects are directed to an (e.g., orbital) implant for correcting a defect of a (e.g., orbital) bone structure. The implant includes a first surface comprising a shape configured to interface with a surface of the bone structure (e.g., a floor and/or a rim of the orbital bone structure). The implant also includes a second surface opposite the first surface and substantially conformal to the shape of the first surface, the second surface comprising three or more point features on the second surface, wherein the three or more point features comprise a first point feature, a second point feature, and a third point feature that form nodes of a triangle, wherein each of the three or more point features are recessed below the second surface.

Certain aspects are directed to a method for correcting a defect of a bone structure. The method includes positioning a first surface of an implant onto the defect of the bone structure, a first surface comprising a shape configured to interface with a surface of the bone structure (e.g., a floor and/or a rim of the orbital bone structure), and a second surface opposite the first surface and substantially conformal to the shape of the first surface, the second surface comprising three or more point features on the second surface, wherein the three or more point features comprise a first point feature, a second point feature, and a third point feature that form nodes of a triangle, wherein each of the three or more point features are at least one of raised above or recessed below the second surface. The method also includes tracing a stylus of a navigation system along the second surface until the stylus is received by cach of the three or more point features to compare one or more of: (i) the shape of the implant relative to a planned shape of the implant, or (ii) a position of the implant in a patient relative to a planned position of the implant in the patient.

Certain aspects are directed to a method for generating an implant for correcting a defect of a bone structure. The method includes receiving a design of the implant, the design indicating a shape, size, and position of the implant relative to a build area corresponding to an additive manufacturing device, the design of the implant defining: a first surface comprising a shape configured to interface with a surface of the bone structure (e.g., a floor and/or a rim of the orbital bone structure); and a second surface opposite the first surface and substantially conformal to the shape of the first surface, the second surface comprising three or more point features on the second surface, wherein the three or more point features comprise a first point feature, a second point feature, and a third point feature that form nodes of a triangle, wherein each of the three or more point features are at least one of raised above or recessed below the second surface. The method also includes causing manufacturing of the implant using additive manufacturing.

### BRIEF DESCRIPTION OF THE DRAWINGS

FIG. 1 is a diagram illustrating an example of a computer environment suitable for the implementation of three-dimensional (3D) object design, build simulation, and manufacturing.
FIG. 2 is a diagram illustrating a functional block diagram of one example of a computer of FIG. 1.
FIG. 3 is a diagram illustrating a process for manufacturing a 3D object or device.
FIG. 4A is a diagram illustrating an exemplary additive manufacturing apparatus for generating a 3D object.
FIG. 4B is a diagram illustrating a recoating mechanism (e.g., a leveling drum/roller) that may be used instead of the recoating mechanism of FIG. 4A.
FIGs. 5A-5E are diagrams illustrating an example orbital implant installed over an orbital floor of a patient's right eye socket.
FIGs. 6A and 6B are diagrams illustrating an example off-the-shelf orbital implant, and the same implant in a bent shape for implantation, according to aspects of the disclosure.
FIGs. 7-23 are diagrams illustrating patient-specific orbital implants according to aspects of this disclosure.
FIG. 24 is a diagram illustrating various examples of elevated ridges according to aspects of this disclosure.
FIG. 25 is a flow diagram illustrating example operations for using an optical implant.
FIG. 26 is a schematic diagram illustrating a conceptual example of a navigation system.

### DETAILED DESCRIPTION

Apparatuses and methods disclosed herein include techniques for producing implants with navigation fiducials, and using the implants for improved surgical implantation and defect resolution procedures. The aspects of the present invention are illustrated by means of orbital implants, but a person skilled in the art will readily appreciate that they also apply to other types of implants, particularly implants that are partially or entirely hidden from view during installation in the patient's body or implants that may be shaped to better match the patient's anatomy. In certain aspects, the orbital implants comprise thin plates (sometimes perforated) or meshes. While features of the present disclosure may be discussed relative to certain embodiments and figures below, all embodiments of the present disclosure can include one or more of the advantageous features discussed herein. In other words, while one or more embodiments may be discussed as having certain advantageous features, one or more of such features may also be used in accordance with various other embodiments discussed herein. In similar fashion, while exemplary embodiments may be discussed below as device, instrument, or method embodiments it should be understood that such exemplary embodiments can be implemented in various devices, instruments, and methods. Unless explicitly mentioned, all method steps disclosed herein may be entirely performed by a computing device, partly performed by a computing device, or partly or entirely performed by a skilled user, such as a medical professional or non-medical professional, such as a technician or engineer. Accordingly, the word "specialist" refers to such a skilled medical or non-medical user. For example, all method steps described for the design and production of implants, including but not limited to image segmentation, defect reconstruction, implant design, implant bending and implant manufacturing, may be entirely performed by a computing device, partly performed by a computing device, or partly or entirely performed by a skilled user, such as a medical professional or non-medical professional, such as a technician or engineer.

In this disclosure, the terms "distal" and "proximal" in the context of an implant refer to positions relative to a specialist placing the implant when the implant is in its planned position with respect to the patient's anatomy. For example, the distal edge of an implant is the edge farthest away from the specialist when the implant is in its planned implanted position. In this disclosure, the terms "anterior" and "posterior" in the context of an implant refer to positions relative to the patient's anatomy as the implant is in its planned position with respect to the patient's anatomy. For example, the anterior edge of an implant is the edge most towards the anterior side of the patient when the implant is in its planned implanted position.

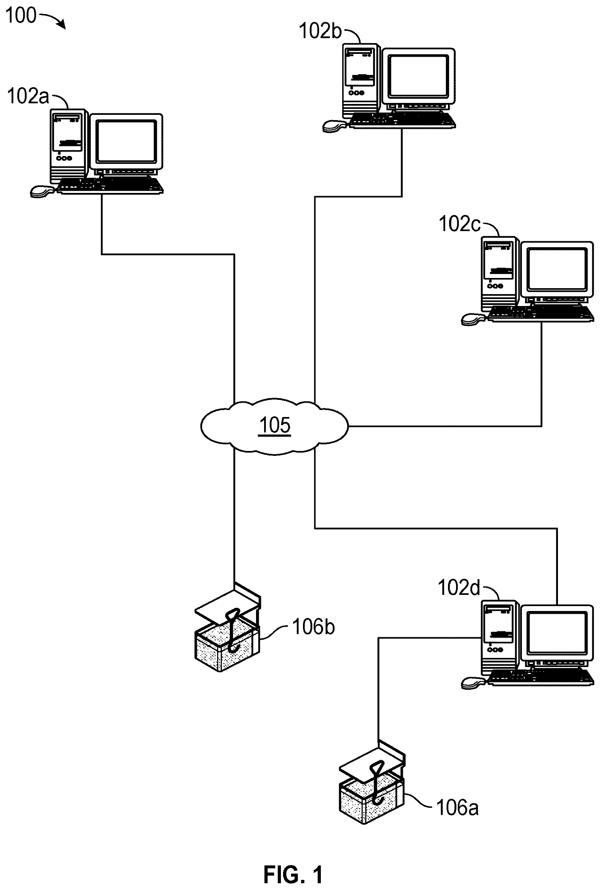

In certain aspects, an orbital implant may be designed on a computing system using any suitable computer-aided design (CAD) software, and/or any suitable system for designing, simulating, and/or manufacturing 3D objects. Turning to FIG. 1, an example of a computer environment suitable for the implementation of 3D object design, build simulation, and manufacturing is shown. The environment includes a system 100. The system 100 includes one or more computers 102a-102d, which can be, for example, any workstation, server, or other computing device capable of processing information. In some embodiments, each of the computers 102a-102d can be connected, by any suitable communications technology (e.g., an internet protocol), to a network 105 (e.g., the Internet). Accordingly, the computers 102a-102d may transmit and receive information (e.g., software, digital representations of three dimensional (3D) objects, commands or instructions to operate an additive manufacturing device, etc.) between each other via the network 105.

The system 100 further includes one or more additive manufacturing devices (e.g., 3D printers) 106a-106b. As shown the additive manufacturing device 106a is directly connected to a computer 102d (and through computer 102d connected to computers 102a-102c via the network 105) and additive manufacturing device 106b is connected to the computers 102a-102d via the network 105. Accordingly, one of skill in the art will understand that an additive manufacturing device 106 may be directly connected to a computer 102, connected to a computer 102 via a network 105, and/or connected to a computer 102 via another computer 102 and the network 105.

It should be noted that though the system 100 is described with respect to a network and one or more computers, the techniques described herein also apply to a single computer 102, which may be directly connected to an additive manufacturing device 106.

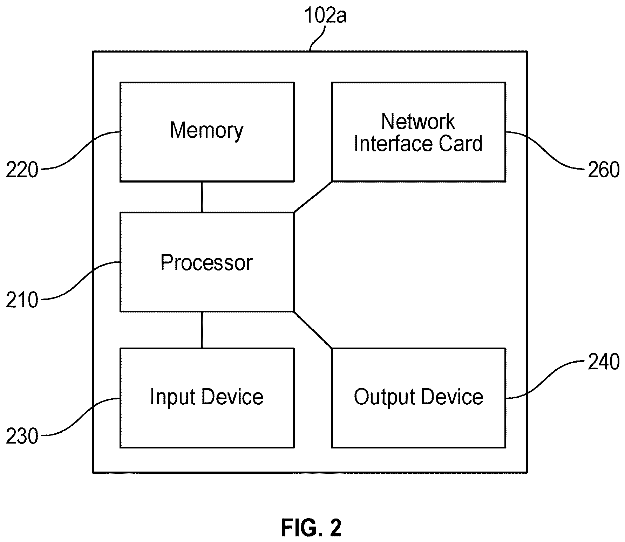

FIG. 2 illustrates a functional block diagram of one example of a computer of FIG. 1. The computer 102a includes a processor 210 in data communication with a memory 220, an input device 230, and an output device 240. In some embodiments, the processor is further in data communication with an optional network interface card 260. Although described separately, it is to be appreciated that functional blocks described with respect to the computer 102a need not be separate structural elements. For example, the processor 210 and memory 220 may be embodied in a single chip.

The processor 210 can be a general purpose processor, a digital signal processor (DSP), an application specific integrated circuit (ASIC), a field programmable gate array (FPGA) or other programmable logic device, discrete gate or transistor logic, discrete hardware components, or any suitable combination thereof designed to perform the functions described herein. A processor may also be implemented as a combination of computing devices, e.g., a combination of a DSP and a microprocessor, a plurality of microprocessors, one or more microprocessors in conjunction with a DSP core, or any other such configuration.

The processor 210 can be coupled, via one or more buses, to read information from or write information to memory 220. The processor may additionally, or in the alternative, contain memory, such as processor registers. The memory 220 can include processor cache, including a multi-level hierarchical cache in which different levels have different capacities and access speeds. The memory 220 can also include random access memory (RAM), other volatile storage devices, or non-volatile storage devices. The storage can include hard drives, flash memory, etc.

The processor 210 also may be coupled to an input device 230 and an output device 240 for, respectively, receiving input from and providing output to a user of the computer 102a. Suitable input devices include, but are not limited to, a keyboard, buttons, keys, switches, a pointing device, a mouse, a joystick, a remote control, an infrared detector, a bar code reader, a scanner, a video camera (possibly coupled with video processing software to, e.g., detect hand gestures or facial gestures), a motion detector, or a microphone (possibly coupled to audio processing software to, e.g., detect voice commands). Suitable output devices include, but are not limited to, visual output devices, including displays and printers, audio output devices, including speakers, headphones, earphones, and alarms, additive manufacturing devices, and haptic output devices.

The processor 210 further may be coupled to a network interface card 260. The network interface card 260 prepares data generated by the processor 210 for transmission via a network according to one or more data transmission protocols. The network interface card 260 also decodes data received via a network according to one or more data transmission protocols. The network interface card 260 can include a transmitter, receiver, or both. In other embodiments, the transmitter and receiver can be two separate components. The network interface card 260, can be embodied as a general purpose processor, a digital signal processor (DSP), an application specific integrated circuit (ASIC), a field programmable gate array (FPGA) or other programmable logic device, discrete gate or transistor logic, discrete hardware components, or any suitable combination thereof designed to perform the functions described herein.

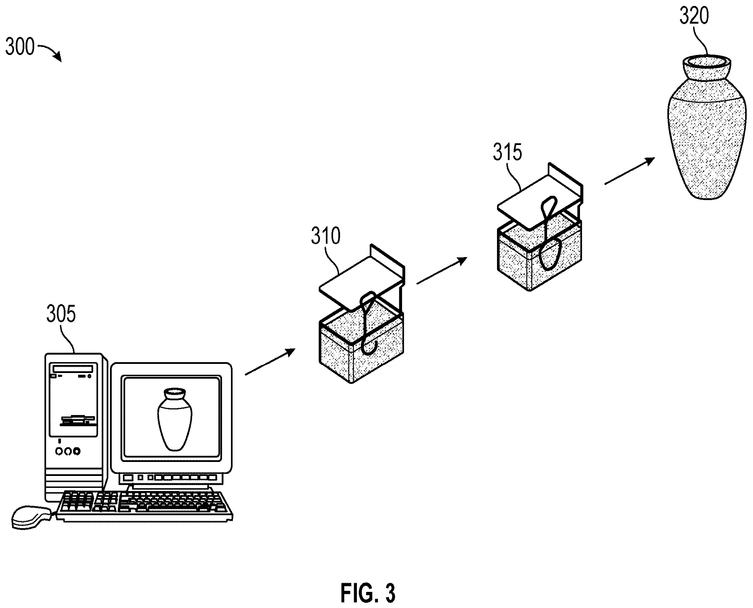

FIG. 3 illustrates a process 300 for manufacturing a 3D object or device. As shown, at a step 305, a digital representation of the object is designed using a computer, such as the computer 102a. For example, two dimensional (2D) or 3D data may be input to the computer 102a for aiding in designing the digital representation of the 3D object. Continuing at a step 310, information corresponding to the 3D object is sent from the computer 102a to an additive manufacturing device, such as additive manufacturing device 106, and the device 106 commences a manufacturing process for generating the 3D object in accordance with the received information. At a step 315, the additive manufacturing device 106 continues manufacturing the 3D object using suitable materials, such as a polymer or metal powder. Further, at a step 320, the 3D object is generated.

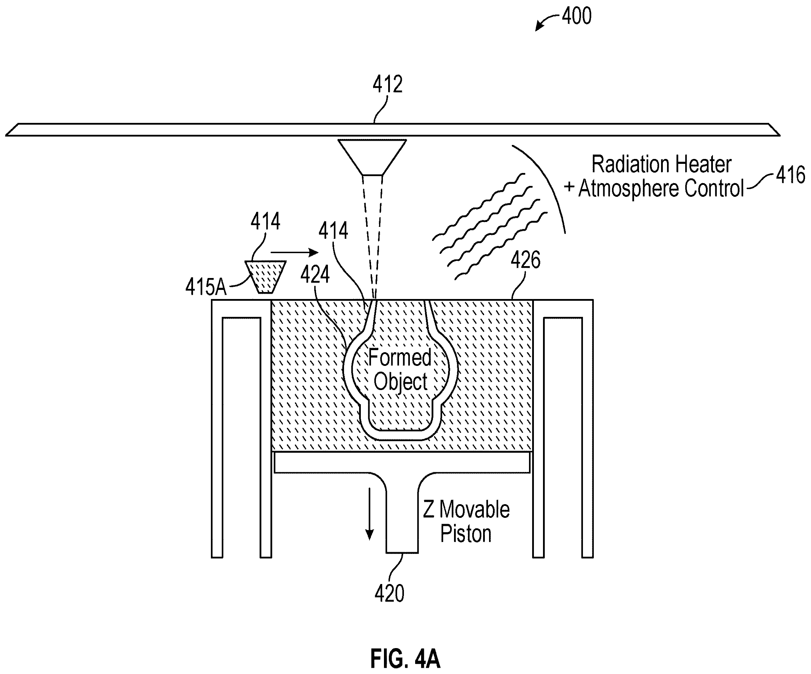

FIG. 4A illustrates an exemplary additive manufacturing apparatus 400 for generating a 3D object. In this example, the additive manufacturing apparatus 400 is a laser sintering device. The laser sintering device 400 may be used to generate one or more 3D objects layer by layer. The laser sintering device 400, for example, may utilize a powder (e.g., metal, polymer, etc.), such as the powder 414, to build an object a layer at a time as part of a build process.

Successive powder layers are spread on top of each other using, for example, a recoating mechanism 415A (e.g., a re-coater blade). The recoating mechanism 415A deposits powder for a layer as it moves across the build area, for example in the direction shown, or in the opposite direction if the recoating mechanism 415A is starting from the other side of the build area, such as for another layer of the build. After deposition, a computer-controlled carbon dioxide (CO2) laser beam scans the surface and selectively binds together the powder particles of the corresponding cross section of the product. In some embodiments, the laser scanning device 412 is an X axis and Y axis moveable infrared laser source. As such, the laser source can be moved along an X axis and along a Y axis in order to direct its beam to a specific location of the top most layer of powder. Alternatively, in some embodiments, the laser scanning device 412 may comprise a laser scanner which receives a laser beam from a stationary laser source, and deflects it over moveable mirrors to direct the beam to a specified location in the working area of the device. During laser exposure, the powder temperature rises above the material (e.g., glass, polymer, metal) transition point after which adjacent particles flow together to create the 3D object. The device 400 may also optionally include a radiation heater (e.g., an infrared lamp) and/or atmosphere control device 416. The radiation heater may be used to preheat the powder between the recoating of a new powder layer and the scanning of that layer. In some embodiments, the radiation heater may be omitted. The atmosphere control device may be used throughout the process to avoid undesired scenarios such as, for example, powder oxidation.

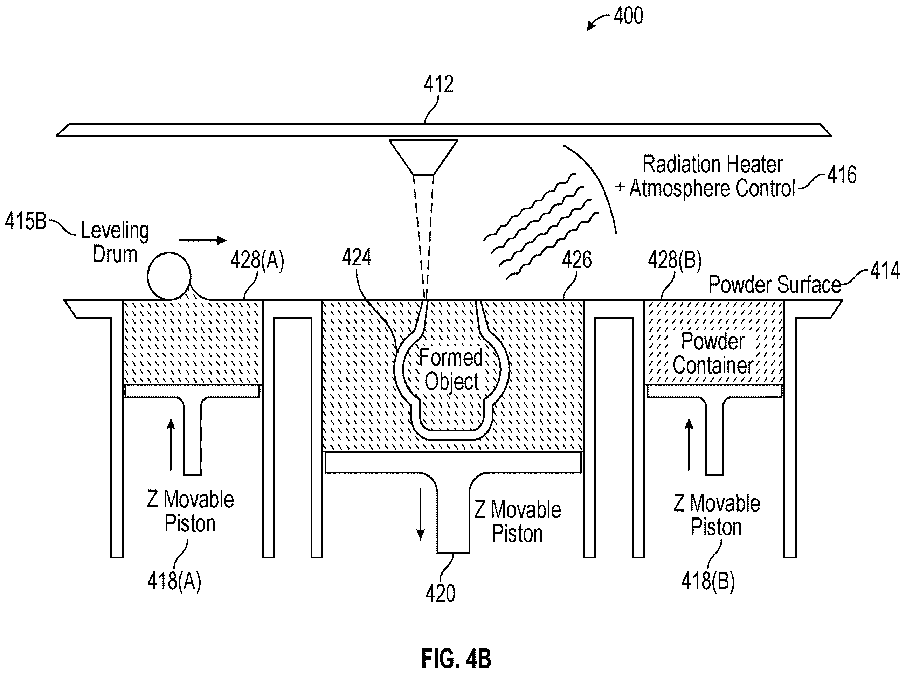

In some other embodiments, such as shown with respect to FIG. 4B, a recoating mechanism 415B (e.g., a leveling drum/roller) may be used instead of the recoating mechanism 415A. Accordingly, the powder may be distributed using one or more moveable pistons 418(a) and 418(b) which push powder from a powder container 428(a) and 428(b) into a reservoir 426 which holds the formed object 424. The depth of the reservoir, in turn, is also controlled by a moveable piston 420, which increases the depth of the reservoir 426 via downward movement as additional powder is moved from the powder containers 428(a) and 428(b) in to the reservoir 426. The recoating mechanism 415B, pushes or rolls the powder from the powder container 428(a) and 428(b) into the reservoir 426. Similar to the embodiment shown in FIG. 4A, the embodiment in FIG. 4B may use the radiation heater 416 alone for preheating the powder between recoating and scanning of a layer.

### EXAMPLE ORBITAL IMPLANTS

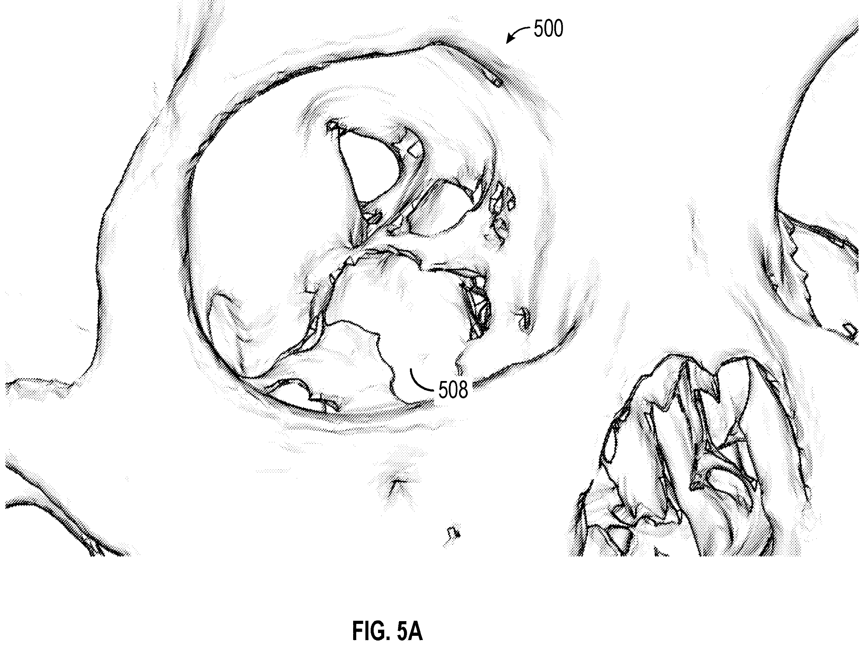

FIGs. 5A-5E are diagrams illustrating various stages of implanting an orbital implant in a patient.

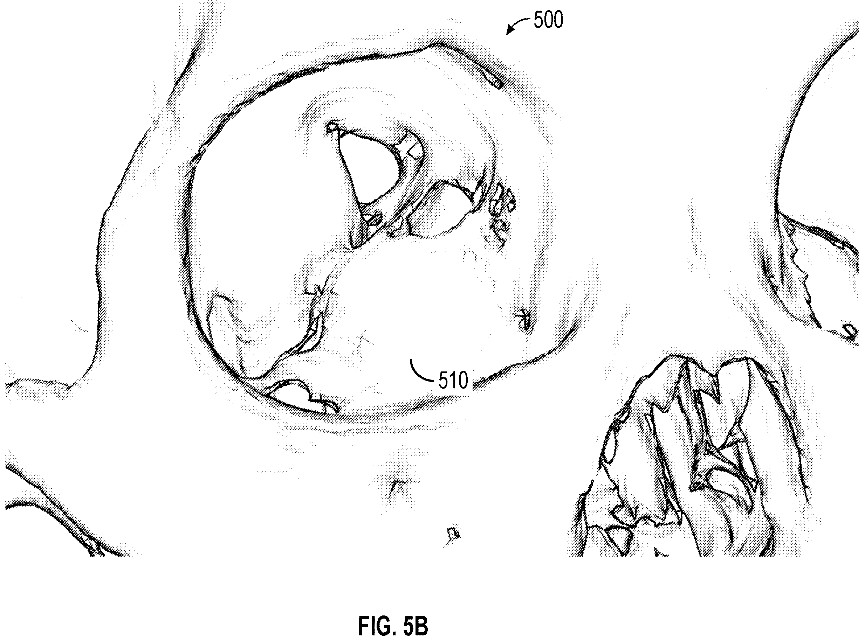

FIG. 5A illustrates an example computer-generated three-dimensional (3D) model of a defect 508 in the orbital floor of a patient's right eye socket 500. A skilled user may generate a model of the defect 508 in order to better model an ideal placement and shape of an implant. For example, FIG. 5B illustrates an example of the model of FIG. 5A, in which the healthy anatomical surface surrounding defect 508 is married with a reconstructed anatomical surface 510 (e.g., wherein the reconstructed anatomical surface 510 is a mirror image of the patient's orbital floor in the left eye socket). This model of a desired bone structure can be used to produce (e.g. by means of additive manufacturing) a physical model of the desired bone structure, which may serve as a bending mold for pre-bending an off-the-shelf implant, or can be used for designing and manufacturing a patient-specific implant.

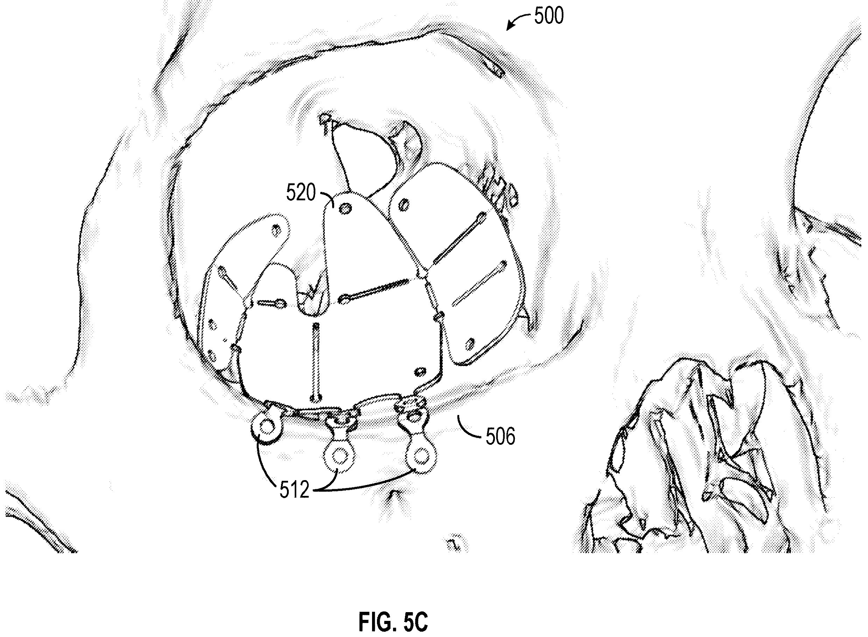

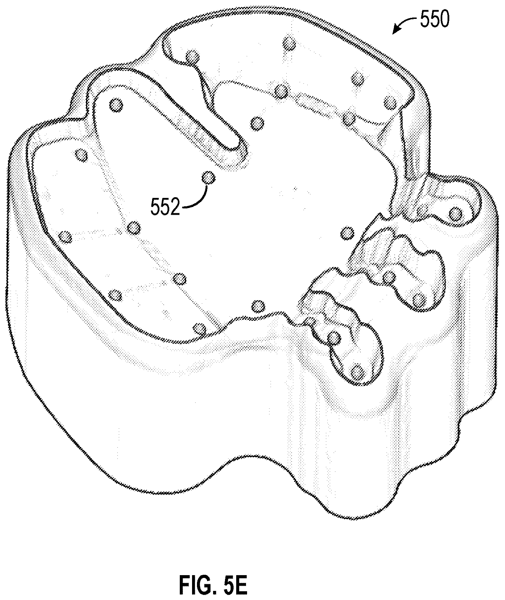

FIG. 5C is a diagram illustrating an example of a model off-the-shelf orbital implant 520 installed over the defect 508 in the orbital floor of the patient's right eye socket 500. Here, the 3D model of FIGs. 5A and 5B provides a basis for determining the extent to which portions of the off-the-shelf orbital implant 520 should be bent or reshaped in order to best imitate the healthy anatomical surface 510 of FIG. 5B. The off-the-shelf orbital implant 520 includes three extensions 512 configured to be attached to a rim 506 of the orbital bone structure by means of fixation elements, such as screws. A 3D model of the bent or reshaped off-the-shelf implant can be used to design and produce a bending mold, such as the one shown in FIG. 5E: FIG. 5E is a diagram illustrating an example bending mold 550 for pre-operatively or intra-operatively bending an off-the-shelf implant.

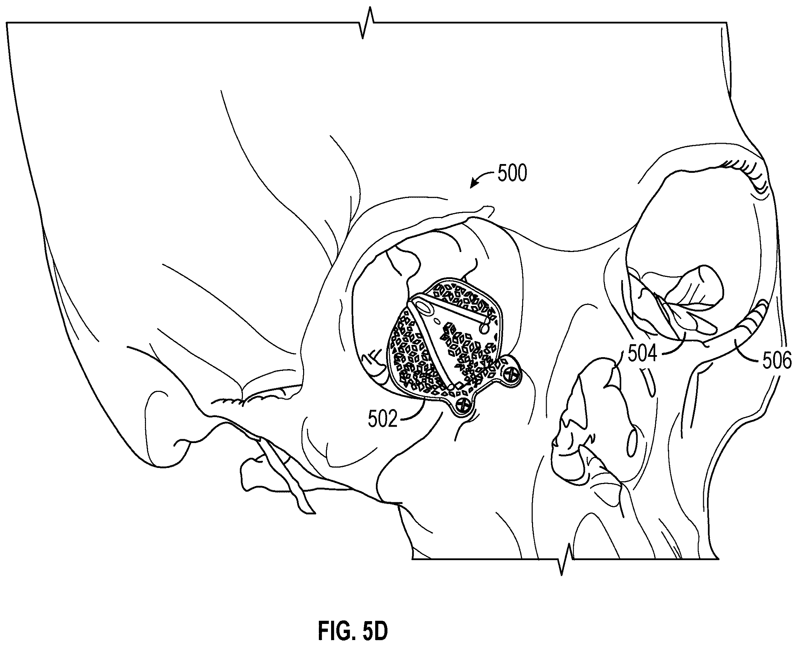

FIG. 5D is a diagram illustrating an example of a model patient-specific orbital implant 502 installed over an orbital floor 504 of a patient's right eye socket 500, and attached to a rim 506 of the orbital bone structure. Here, the 3D model of FIGs. 5A and 5B provides a basis for designing a shape of the patient-specific orbital implant in order to best imitate the healthy anatomical surface 510 of FIG. 5B.

As discussed, patient-specific orbital implants 502 may be designed based on information about the patient's orbital bone structure. Such information can be found in, for example, computerized tomography (CT) scans, magnetic resonance imaging (MRI) scans, ultrasound images, or images from other suitable medical imaging modalities. Through a process called "segmentation," the information in these medical images can be used to develop a virtual 3D model of the patient's orbital bone structure (e.g., by means of the Materialise Mimics software). With the use of CAD software, the orbital implant 502 can be specifically designed to mate with a portion of the patient's orbital bone structure to resolve a structural defect. The orbital implant 502 can then be manufactured by using the additive manufacturing technology discussed above, such as selective laser sintering or selective laser melting with any suitable material, such as titanium, titanium alloy, stainless steel, polyether ether ketone (PEEK), etc.

In some examples, a defect in an orbital bone structure may prevent a specialist from developing a 3D model that accurately models the desired bone structure. In such a case, the specialist may instead model an orbital bone structure of a contralateral side if that side is intact. Thus, a mirror image of at least a part of the virtual 3D model containing the contralateral eye socket may be registered with the healthy tissue surrounding the defect and used as a template for the design of the orbital implant 502. Alternatively, a statistical shape model (SSM) of the orbital bone structure may be used to model the orbital bone structure based on the healthy or intact tissue and/or bone surrounding the defect. For example, an SSM may be constructed based on segmentation or partitioning of a digital image or stack of digital images into multiple segments (e.g., sets of pixels or voxels). Next, the segmentation changes the representation of the image into something relatively easier to analyze, such as a three-dimensional model such as a wireframe model or a surface model, enveloping one or more of such sets of pixels or voxels. In some examples, the digital images are analyzed to locate objects and/or boundaries, such as anatomical areas of interest. In this case, the anatomical area of interest may include the orbital bone structure. Based on a statistical analysis of a training set (e.g., a number of such three-dimensional models from specimens of a population), an SSM may be created, which is a three-dimensional deformable representation that encodes the correlations between shape variations among members of training set, which is assumed to be representative for the entire population. An SSM is characterized by a vector of limited length. By assigning values to the coordinates of this vector, the SSM may be deformed in a way that is consistent with the shape variations witnessed in the training set. Once an SSM is created based on a training set of intact orbital bones, the SSM can be fitted to the healthy or intact tissue and/or bone surrounding the defect. The instance of the SSM thus obtained may then comprise a section spanning the defect and exhibiting a shape representative of non-defective tissue. It may therefore be used to model a desired bone structure (i.e. reconstruct the defect). The desired bone structure may be used as a template for designing the orbital implant 502. The benefit of such a process is that it increases the likelihood of the eyeball being pushed back in its original position, which is one of the main benefits of patient-specific orbital implants 502. In some aspects, the present disclosure relates to a method as described above for designing and/or manufacturing any of the patient-specific implant described below.

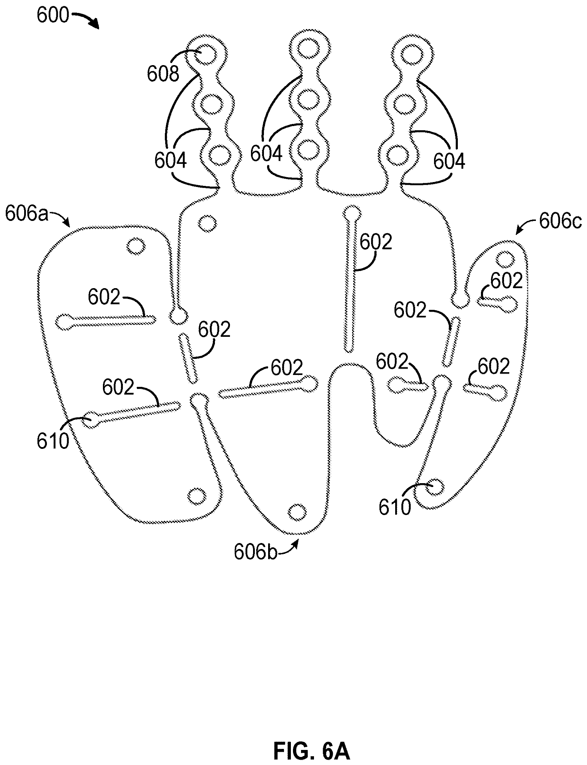

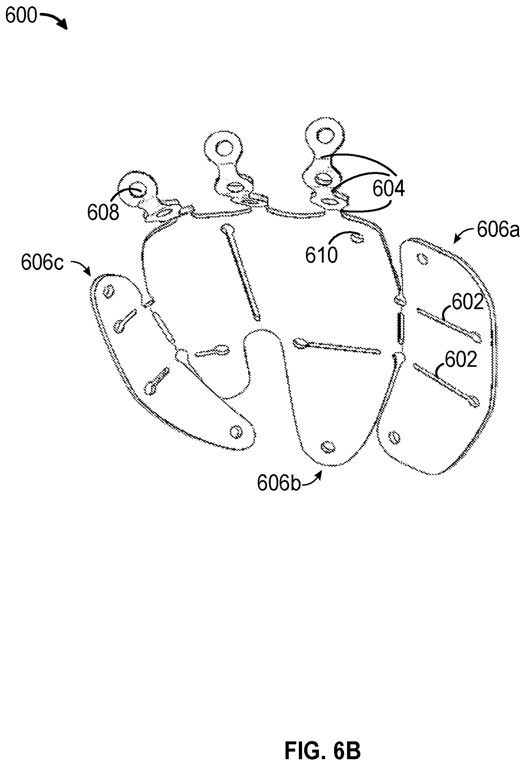

Pre-operative planning can transfer many of the advantages of patient-specific orbital implants 502 to off-the-shelf implants (e.g., see the off-the-shelf orbital implant 600 described in more detail in FIGs. 6A and 6B below). For example, information about the patient's bony anatomy may be obtained (step 1) from medical images and used to develop (step 2) a 3D model (e.g., see FIG. 5A) as described above (e.g., model of orbital bone structure). A target shape for an eyeball-facing surface of the orbital implant may then be designed (step 3) using the 3D model, a mirror image of the contralateral side, and/or an SSM to model a desired bone structure as described above. The target shape may then be offset towards the bone over a distance equal to the thickness of the implant. The target shape or the resulting offset target surface may then be married to the 3D model of the patient's bone structure surrounding the defect to obtain (step 4) a 3D model of a desired bone structure (e.g., see FIG. 5B) to serve as a bending mold. This bending mold, i.e. a physical model of the desired bone structure, may be manufactured (step 5) from the 3D model using an additive manufacturing technology in any suitable material, such as polyamide. A skilled user, such as a surgeon or a non-medical professional, such as a technician or an engineer, may then use this mold to select the most appropriate off-the-shelf implant (e.g., see FIG. 6A) type and size, and bend (step 6 - see FIG. 5C) the structure of the selected implant to fit the patient anatomy and the target shape. In some aspects, the present disclosure relates to a kit of such a bending mold and an off-the-shelf implant. In some aspects, the present disclosure relates to a method as described above for designing and/or manufacturing a physical model of the desired bone structure to serve as a bending mold as described above and/or for selecting and/or bending an off-the-shelf implant.

In some examples, the bending of the selected off-the-shelf orbital implant may first be simulated using a 3D model of the selected off-the-shelf implant and the 3D model of the patient's bone structure. The bending may be simulated such that the eyeball-facing surface of the orbital implant closely follows the target shape, and such that the bone-facing surface of the implant is well seated on the bone structure surrounding or adjacent to the defect (e.g., see FIG. 5C). Simulated bending may allow the specialist, such as a medical professional or a non-medical professional, such as a technician or engineer, to choose an appropriate position for the implant. This can ensure that fixation points (e.g., attachment points where the orbital implant is attached to the bone structure) are in locations with good bone stock, and that the implant is not in a position where it could disturb or harm delicate anatomical features, such as nerves. Simulated bending may allow said specialist to ensure that the defect is well covered, and that there is little excess implant material. In some examples, a simulation of bending the orbital implant may include a selection of the most appropriate implant type, shape, and/or size from a library.

In some examples, the resulting shape of a virtually bent orbital implant may be used to generate a set of instructions for a sheet-metal bending machine configured to bend the implant according to the instructions. Alternatively, the resulting shape may be used to design a bending mold (e.g., see FIG. 5E) mating with either the eyeball-facing surface or the bone-facing surface of the simulated bend of the implant. For example, the bending mold may have any shape suitable for easy manipulation, such as a prismatic shape with a flat lower surface, and may have a top surface configured to mate with either the eyeball-facing or the bone-facing surface of the virtually bent implant. The bending mold may include markings to delineate the outline of the bent implant, or protrusions 552 to be received in one or more perforations of the implant or notches along the edge of the implant such as the apertures of the mesh or any apertures for receiving fixation elements. The bending mold may be manufactured from the design using the additive manufacturing described above using any suitable material, such as polyamide. A skilled user can then bend the implant until it fits on the bending mold (see FIG. 5E), using any markings and protrusions as aides. The bent implant may then be shipped to the hospital, optionally together with the mold. Alternatively, the mold may be shipped to the hospital, together with the unbent implant. The surgeon or a technician can then bend the implant until it fits on the bending mold, using any markings and protrusions as aides. In some aspects, the present disclosure relates to a kit of such a bending mold and an off-the-shelf implant, either in its original state or in a pre-bent state. In some aspects, the present disclosure relates to methods as described above for selecting and/or virtually bending an off-the-shelf implant, for providing a bending mold and/or for bending an off-the-shelf implant.

Whether an orbital implant is manufactured directly in a patient-specific form or bent according to a pre-operative planning procedure using an off-the-shelf implant, correct positioning of the implant in the patient is important for restoring the natural position of the eyeball. The shape and accessibility of the eye socket is such that it is often only possible to fixate the orbital implant to the bone on one side of the implant, generally along a proximal edge of the implant. A slight deviation from the planned position along this edge may lead to a large deviation along the eyeball-facing surface and the distal edge of the implant. Thus, in some cases, navigation systems may be used during a surgical procedure to verify the position of the orbital implant with respect to the patient anatomy, and to compare this position with one or more of a planned position or with a planned target implant shape.

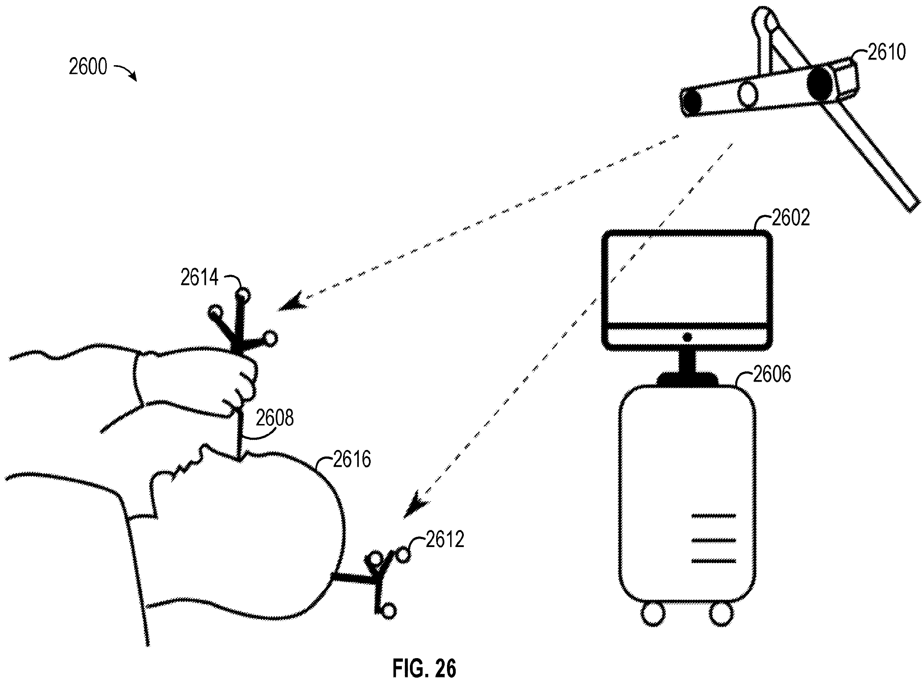

Surgical navigation systems (e.g. see FIG. 26) may provide functionality, including: (i) loading (e.g. onto electronic processing device 2606) and displaying (e.g. on display unit 2602) images or image feeds from intra-operative imaging equipment (e.g., digital/film camera, radiography, magnetic resonance imaging (MRI), computed tomography (CT), fluoroscopy, ultrasound, echocardiography, nuclear medicine, such as positron emission tomography (PET), etc.); (ii) loading (e.g. onto electronic processing device 2606) and displaying (e.g. on display unit 2602) pre-operatively made virtual 3D models of patient anatomy; (iii) loading (e.g. onto electronic processing device 2606) and displaying (e.g. on display unit 2602) pre-operatively made virtual 3D models of any hardware, such as implants; (iv) tracking (e.g. using infra-red sensor 2610) the location and orientation of instruments (e.g., stylus 2608) to determine their position in the navigation system's coordinate system; (v) sweeping surfaces of the anatomy or indicating certain anatomical landmarks with a tracked instrument, such as a probe or a stylus (e.g. stylus 2608) to determine their position in the navigation system's coordinate system; (vi) sweeping surfaces of any hardware, such as implants, or indicating identifiable features (e.g., navigation fiducials) of such hardware, such as points, lines, curves, contours, with a tracked instrument, such as a probe or a stylus (e.g. stylus 2608) to determine their position in the navigation system's coordinate system; and (vii) establishing a relationship between loaded virtual 3D models and (a) images or image feeds from intra-operative imaging equipment, and/or (b) data gathered with tracked instruments to correctly place the virtual 3D models in the navigation system's coordinate system. For example, the information gathered under (v) may be used to locate the patient in the navigation system's coordinate system and/or to bring the patient and pre-operatively made virtual 3D models of patient anatomy into a common coordinate system. For example, a relationship may be established between a "real" coordinate system and a "virtual" coordinate system, and one way of doing this is by sweeping surfaces of the patient's anatomy to establish a correspondence between the patient's anatomy and a loaded pre-operatively made virtual 3D model of the patient's anatomy.

As discussed, the 3D model may be generated from medical images using topological and geometrical information gleaned from the images. In some examples, the relationship between the coordinate system of the 3D model and the real coordinate system is established by matching corresponding anatomical landmarks in both the patient and the virtual 3D model. In other examples, the relationship between the coordinate system of the 3D model and the real coordinate system is established by generating a trajectory along an axis of the geometric model that matches the same trajectory along an axis of the patient's anatomy, and conducting moving trihedron modeling along the trajectory.

Once the patient has been located in the navigation system's coordinate system, any further movement of the patient with respect to the navigation system may be tracked by means of a rigidly attached tracker 2612 attached to the patient anatomy 2616, and optionally another tracker 2614 attached to the stylus 2608 and the spatial correspondence between the actual patient and the virtual 3D model of the anatomy may be maintained. For example, the information gathered under (vi) may be used to locate any hardware in the navigation system's coordinate system and/or to bring the hardware and pre-operatively made virtual 3D models of the hardware into a common coordinate system. For example, the same sweeping procedure discussed above may be performed to establish a relationship between a 3D model of an orbital implant (and a corresponding coordinate system) with the actual physical orbital implant.

Thus, surgical navigation systems may be used to verify the position of an orbital implant during a procedure to install the implant within the patient (e.g., a virtual 3D model of the patient anatomy and a virtual 3D model of the orbital implant (e.g., either in patient-specific form, or off-the-shelf form with or without a planned simulated bend) in its planned position relative to the virtual 3D model of the patient anatomy). The virtual 3D model of the patient anatomy may be used to correctly position the 3D model of the orbital implant in the navigation system's coordinate system.

In some examples, a specialist may use a tracked instrument of a navigation system, such as a probe or a stylus, to trace particular features of the orbital implant. By tracing the features of the implant, the position of these features can be collected and the location of the features in the navigation system's coordinate system may be determined and displayed on the navigation system. By locating the features of the actual implant in a coordinate system, the position of the implant may be compared to a planned position (e.g., a position of the 3D model of the orbital implant relative to the 3D model of the patient anatomy). The navigation system can measure any deviation between the actual physical position of the implant and the planned position of the implant, and report the deviation to the specialist, optionally with instructions on how to modify the position of the implant to match the planned position.

Accordingly, a surgical navigation system may determine an extent to which the planned bending of an optical implant has been achieved, and/or an extent to which the location of the optical implant matches the planned position, by utilizing identifiable features positioned at certain locations of the implant to establish the implant's position in space.

### EXAMPLE TECHNIQUES FOR MANUFACTURING AND USING ORBITAL IMPLANTS WITH NAVIGATION FIDUCIALS

FIG. 6A is a diagram illustrating an example off-the-shelf orbital implant 600 according to aspects of the disclosure. The desired shape of an off-the-shelf implant 600 may be determined either pre-operatively or intra-operatively, and the implant may be bent pre-operatively or intra-operatively to fit the patient anatomy. Any of the bending molds as described above may be used while bending the implant.

As shown, the off-the-shelf orbital implant 600 includes bending locations in the form of linear slots 602 that perforate the implant, or narrowed connections 604. Such bending locations locally reduce the implant's 600 resistance to deformation, while also subdividing the implant into several major sections 606 (e.g., a first major section 606a, a second major section 606b, and a third major section 606c). Here, the narrowed connections 604 connect multiple holes 608 through which the implant 600 can be fastened to a patient's bone structure.

The orbital implant 600 also includes a plurality of point features 610 that a specialist can use to assess a location of the implant 600 in a coordinate system. For example, the point features 610 may be configured to receive a stylus so that the stylus can feedback location information to the navigation system. In some examples, the point features 610 may be positioned on a section's surface and/or along its edges, such as the edges it shares with adjacent sections. In certain aspects, the point features 610 may include a hole at an end of a linear slot 602. This may allow a specialist using the stylus to trace the linear slot 602 to the point feature 610. In some examples, the linear slot 602 may be a path feature that provides the stylus location information in addition to the point feature 610. FIG. 6B illustrates an example bend of the orbital implant 600 of FIG. 6A, wherein the implant 600 is bent to imitate a healthy anatomical orbital floor of a patient.

By locating the features of the actual implant in a common coordinate system with the patient anatomy, the position of the implant may be compared to a planned position (e.g., a position of the 3D model of the orbital implant relative to the 3D model of the patient anatomy). The navigation system can measure any deviation between the actual physical position of (a feature or portion of) the implant and the planned position of (said feature or portion of) the implant, and report the deviation to the specialist, optionally with instructions on how to modify the position of the implant to match the planned position and/or to perform additional bending of the implant to match the planned implant shape. Alternatively, a desired anatomical shape may be reconstructed pre-operatively as described above (e.g. by utilizing a mirror image of the intact contralateral orbital cavity, or an SSM of intact orbital cavities). By intra-operatively locating features of the implant as preliminarily placed on the patient, the navigation system may compare the position of these features with the desired anatomical shape of the orbital cavity, and report the deviation to the specialist, optionally with instructions on how to modify the position of the implant and/or to perform additional bending of the implant to better match the desired anatomical shape of the orbital cavity.

Typically, patient-specific implants are designed specifically for individual patients and are manufactured according to a design developed pre-operation. Although any number of point features may be used, patient-specific implants may require fewer point features relative to off-the-shelf orbital implants because fewer or no implant shape adjustments may be required by a patient-specific implant.

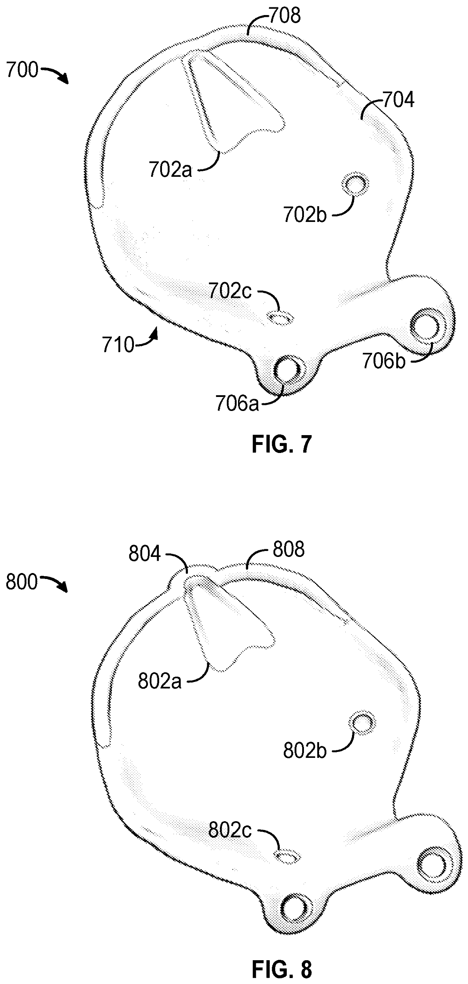

FIG. 7 is a diagram illustrating a patient-specific orbital implant 700 according to aspects of this disclosure. As illustrated, the top surface of the implant 700 is an eyeball-facing surface 704, with a bone-facing surface 710 on the opposite side of the implant 700. The bone-facing surface 710 is configured to interface with a portion of the patient's anatomy (e.g. a portion of the floor and/or anterior rim of the orbital cavity). To this end, the bone-facing surface 710 may be partly or in its entirety shaped to mate with a surface of the of the patient's anatomy. Holes (e.g., a first hole 706a and a second hole 706b) are provided on a proximal edge of the implant 700 to accommodate screws or other attaching elements configured to fix the implant 700 to the bone structure of an eye socket. The implant 700 includes an elevated ridge 708 along a distal edge of the implant 700. The implant 700 also includes three point features (e.g., a first point feature 702a, a second point feature 702b, and a third point feature 702c), in this example in a non-isosceles triangle formation. In some examples, and to increase robustness of implant-position determination, point features may be spread as widely apart as possible.

In this example, the second point feature 702b and the third point feature 702c take the form of dimples, or recesses, in the eyeball-facing surface. These two point features are near the proximal edge of the implant 700, and thus, are easier to reach with the tracked stylus compared to the first point feature 702a which is closer to the distal edge of the implant 700. Here, when using a navigation system to determine a location of the implant 700, a specialist may insert a distal tip of a stylus of the navigation system into the point features 702 to map the implant's location.

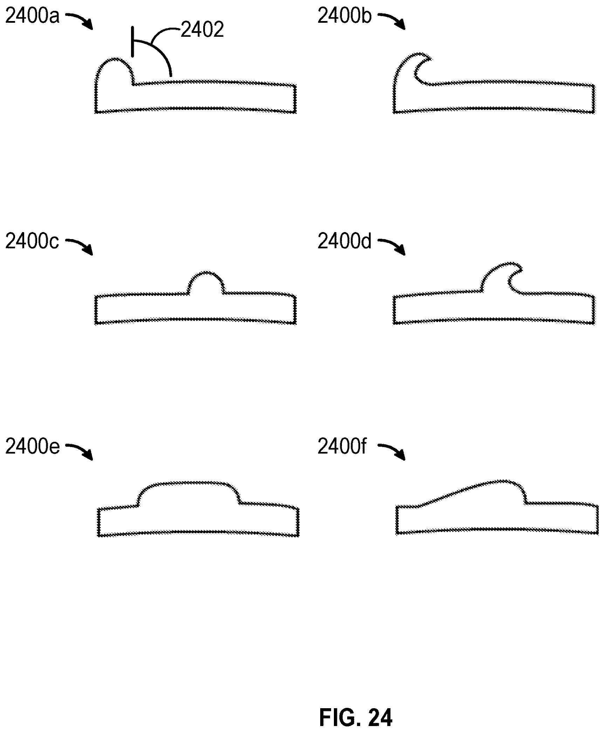

As noted, the first point feature 702a is located closer to the distal edge of the implant 700, and is thus further away from the surgeon implanting the implant 700. During implantation, the distal edge of the implant 700 may sit underneath soft tissue and the eyeball, which may obscure the first point feature from the surgeon's vision. Thus, in order to guide the stylus, the elevated ridge 708 is configured to direct the stylus into the first point feature 702a. In this example, the elevated ridge 708 has at least one side wall that sits at an angle 2402 from the eyeball-facing surface. In some examples, the angle may be greater than 45°, (e.g., an angle between 60° and 90°, see the first cross section 2400a of FIG. 24). The height of the elevated ridge 708 may be high enough to prevent the tip of the stylus from slipping over the ridge, but low enough so as not to cause irritation of the soft tissue covering the implant 700 while in an implanted state (e.g., a height between 0.5mm and 1.5mm). As discussed below, the elevated ridge 708 may include one or more other shapes and sizes.

To verify the position of the implant intra-operatively, the surgeon may place the tip of the stylus in the second point feature 702b and the third point feature 702c to indicate the proximal point features. The surgeon may also trace the tip of the stylus over the eyeball-facing surface in a distal direction until it hits the elevated ridge 708. By then sliding the stylus in a lateral direction along the elevated ridge 708, the surgeon can guide the stylus tip to the first point feature 702a. Accordingly, no visual access of the distal region of the implant is necessary.

The stylus may include an angled tip, or alternatively, a straight tip. However, a stylus with a straight tip may have difficulties entering into a dimple or hole, because of the narrow access between the implant and the soft tissue and because of its consequently sharp angulation with respect to the eyeball-facing surface. Accordingly, the stylus may need to be held in a position close to tangential to the surface (e.g. at an angle between the shaft of the stylus and the surface of 30 degrees or less). Thus, as illustrated in FIG. 7, the first point feature 702a is a wedge-shaped depression that opens towards the proximal side of the implant 700 to be able to receive the stylus. While pushing the tip of the stylus along elevated ridge 708, the surgeon may sense that the stylus drops into the depression. Thus, once in the depression, the surgeon may continue to push the stylus in a distal direction to keep the tip of the stylus in the apex of the depression until the stylus meets the elevated ridge 708. It should be noted that any of the point features described herein may include one or more of a dimple, a depression, a hole, or any other suitable physical marking in the eyeball-facing surface to indicate that the stylus has reached a particular point or location of the implant. In certain embodiments, at least three point features may be used, as three points may be sufficient to accurately locate the implant in a coordinate system. In certain aspects, the three points form a triangle (such as, a non-isosceles triangle) such that each edge length between the points is different so that each edge can be differentiated to better determine a position and orientation of the implant in a coordinate system.

FIG. 8 is a diagram illustrating a patient-specific orbital implant 800 according to aspects of this disclosure. The implant 800 includes several characteristics that are similar to those shown in FIG. 7, including a first point feature 802a located near a distal edge of the implant 800, and a second point feature 802b and a third point feature 802c near a proximal edge. However, in this example, an elevated ridge 808 at the distal end of the implant 800 includes a notch 804 partially surrounding the first point feature 802a. The notch 804 may be configured to indicate to the surgeon manipulating the stylus when the tip of the stylus has reached the first point feature 802a in addition to the depression of the first point feature 802a. In some examples, the notch 804 may be used as an alternative to the depression characteristic. As an alternative to the notch 804, an interruption of the elevated ridge 808 may be used. For example, the elevated ridge 808 may include a gap or a notch configured to act as a first point feature, wherein the gap is smaller than the diameter of the tip of the stylus so that it "catches" the stylus.

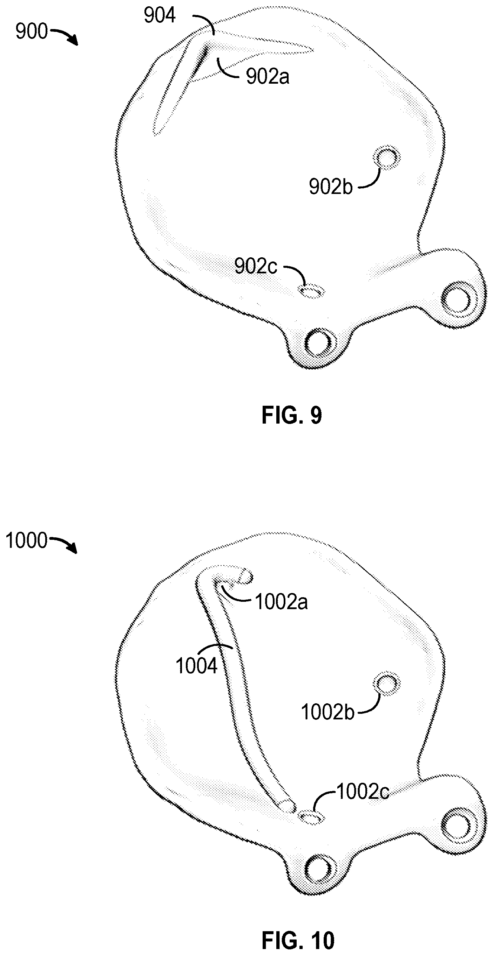

FIG. 9 is a diagram illustrating a patient-specific orbital implant 900 according to aspects of this disclosure. The implant 900 includes several characteristics that are similar to those shown in FIGs. 7 and 8, including a first point feature 902a located near a distal edge of the implant 900, and a second point feature 902b and a third point feature 902c near a proximal edge. In this example, the implant 900 includes an elevated ridge 904 that does not follow the distal edge of the implant 900, but rather follows a V-shaped trajectory. Here, when a surgeon pushes the stylus against the elevated ridge 904, it will cause the stylus to be guided towards the V-shape's apex, where the first point feature 902a is located. The surgeon may sense the change in direction of the elevated ridge 904 and understand that the first point feature 902a has been reached. If more than one point feature is arranged along the elevated ridge 904, the elevated ridge's trajectory may be shaped as a concave and/or polyline, with segments (e.g., straight elevated ridges) between the point features. The first point feature 902a may optionally include a depression region to help "catch" the stylus. As in the examples above, the elevated ridge may include a notch at least partially surrounding the first point feature 902a, or an interruption at the first point feature 902a.

FIG. 10 is a diagram illustrating a patient-specific orbital implant 1000 according to aspects of this disclosure. The implant 1000 includes several characteristics that are similar to those shown in FIGs. 7-9 that may not be discussed further for the purposes of brevity. In this example, an elevated ridge 1004 does not run near the distal edge of the implant 1000 as in the previous examples, but from a third point feature 1002c to a first point feature 1002a. After indicating the third point feature 1002c (e.g., a point feature that is visible to the surgeon) with the stylus, the surgeon may slide the tip of the stylus along the elevated ridge 1004 until it reaches the first point feature 1002a. The elevated ridge 1004 has a hook shape or hockey-stick shape that curves around the first point feature 1002a to trap the stylus at the first point feature 1002a and signal to the surgeon when the stylus has reached that point feature.

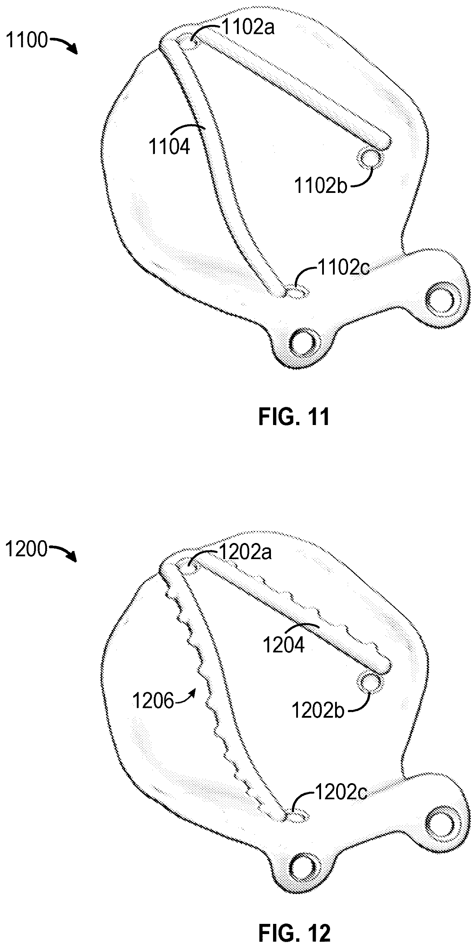

FIG. 11 is a diagram illustrating a patient-specific orbital implant 1100 according to aspects of this disclosure. The implant 1100 includes several characteristics that are similar to those shown in FIGs. 7-10 that may not be discussed further for the purposes of brevity. Here, the implant 1100 includes an elevated ridge 1104 that follows a path between a third point feature 1102c and a first point feature 1102a. The elevated ridge 1104 also has a second leg continuing from the first point feature 1102a to a second point feature 1102b. Because the elevated ridge 1104 is continuous, the stylus may be guided through the entire sequence of point features.

FIG. 12 is a diagram illustrating a paticnt-spccific orbital implant 1200 according to aspects of this disclosure. The implant 1200 includes several characteristics that are similar to those shown in FIGs. 7-11 that may not be discussed further for the purposes of brevity. Here, the implant 1200 includes an elevated ridge 1204 to guide the stylus to one or more point features. However, because the point features are arranged to one side of the elevated ridge 1204, the elevated ridge 1204 includes a series of alternating indentations and protrusions 1206 configured to notify the surgeon when the stylus is along the wrong side of the elevated ridge 1204 by preventing the stylus from moving and/or making progression of the stylus more difficult along that side of the ridge 1204. Such warning features may be combined with the elevated ridges of any of the other examples.

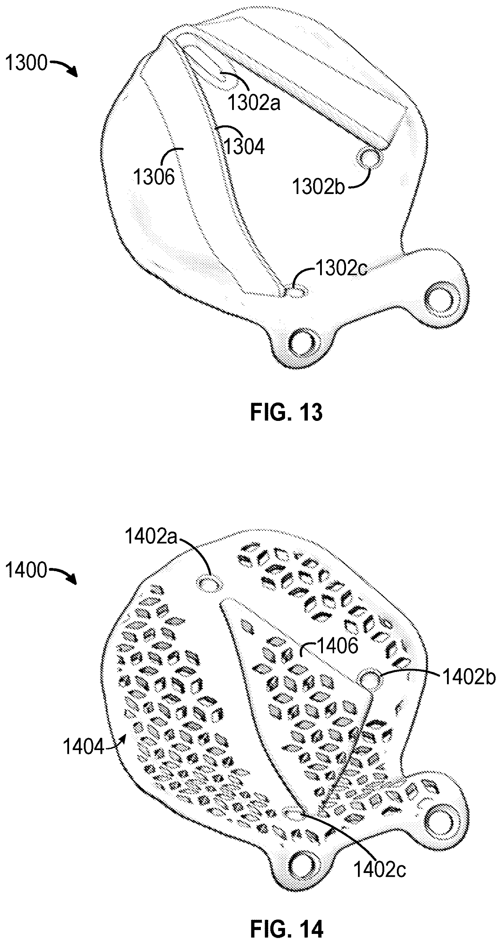

FIG. 13 is a diagram illustrating a patient-specific orbital implant 1300 according to aspects of this disclosure. The implant 1300 includes several characteristics that are similar to those shown in FIGs. 7-12 that may not be discussed further for the purposes of brevity. Here, a first point feature 1302a is a depression at an apex of an elevated ridge 1304. The depression has an elongated shape so as to receive the tip of the stylus held in a position close to tangential to the eyeball-facing surface. The elevated ridge 1304 includes a side that makes a smooth or gradual transition 1306 from the top of the elevated ridge 1304 to the surface of the implant 1300. The smooth transition 1306 prevents the stylus being guided along the wrong side of the elevated ridge, and instead allows the stylus to be guided back to the correct side without having to lift the stylus from the surface of the implant 1300. Such a smooth transition may be combined with the elevated ridges of any of the other examples.

FIG. 14 is a diagram illustrating a paticnt-spccific orbital implant 1400 according to aspects of this disclosure. The implant 1400 includes several characteristics that are similar to those shown in FIGs. 7-13 that may not be discussed further for the purposes of brevity. Here, the implant has an elevated ridge 1406 configure to guide a stylus toward point features 1402. In contrast to previous examples, the elevated ridge 1406 is positioned inside of a triangle formed by the point features 1402. In this example, the elevated ridge 1406 forms a plateau 1406 in the shape of a triangle. However, in some examples, the center of the triangle may include an elevation that is the same as the surface of the implant 1300 outside of the triangle. The implant 1400 also includes a plurality of perforations 1404 that pass through the eyeball-facing surface to the opposite side. The size of the perforations 1404 may be relatively smaller than the point features 1402 and/or the stylus to prevent the surgeon from wrongly indicating point features. Note that in this example there are no perforations 1404 along paths between point features 1402. This prevents the stylus from being caught by a perforation or falsely indicating a point feature 1402. Such perforations 1404 may be combined with any of the other examples. The plateau-style elevated ridge 1406 may also be used without perforations 1404.

The following examples demonstrate how features described above may be freely combined. They are not limitative to the scope of the present disclosure.

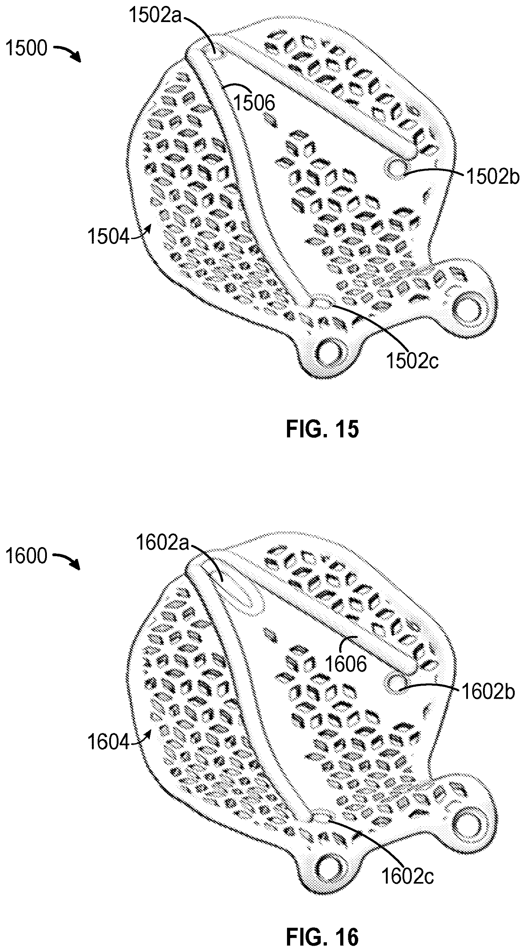

FIG. 15 is a diagram illustrating a patient-specific orbital implant 1500 according to aspects of this disclosure. The implant 1500 includes several characteristics that are similar to those shown in FIGs. 7-14 that may not be discussed further for the purposes of brevity. Here, the implant 1500 includes perforations 1504 and an elevated ridge 1506 configured to guide a stylus between point features 1502.

FIG. 16 is a diagram illustrating a paticnt-spccific orbital implant 1600 according to aspects of this disclosure. The implant 1600 includes several characteristics that are similar to those shown in FIGs. 7-15 that may not be discussed further for the purposes of brevity. Here, the implant 1600 includes perforations 1604 and an elevated ridge 1606 configured to guide a stylus between point features 1602. In this example, a first point feature 1602a is a depression, while a second point feature 1602b and a third point feature 1602c are holes or dimples.

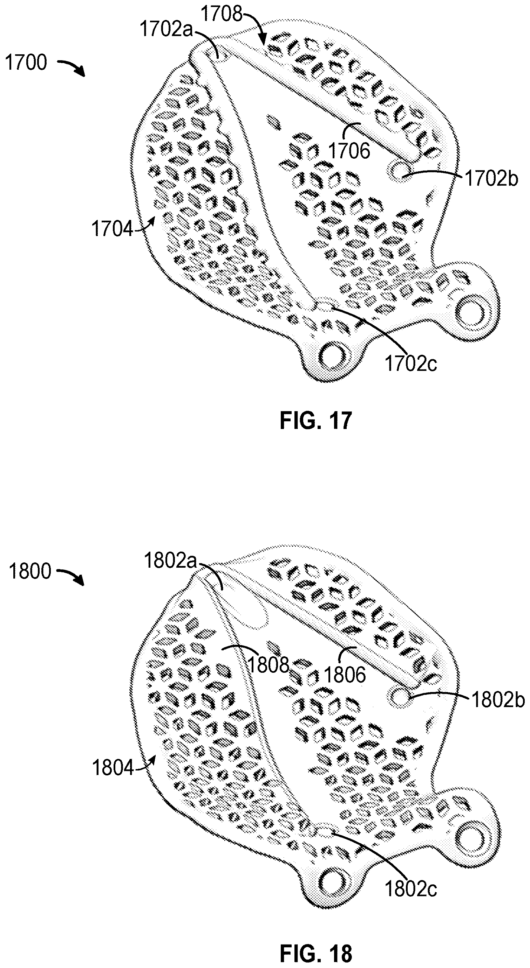

FIG. 17 is a diagram illustrating a patient-specific orbital implant 1700 according to aspects of this disclosure. The implant 1700 includes several characteristics that are similar to those shown in FIGs. 7-16 that may not be discussed further for the purposes of brevity. Here, the implant 1700 includes perforations 1704 and an elevated ridge 1706 configured to guide a stylus between point features 1702. Because the point features are arranged to one side of the elevated ridge 1706, the elevated ridge 1706 includes a series of alternating indentations and protrusions 1708 configured to notify the surgeon that the stylus along the wrong side of the elevated ridge 1706 by preventing the stylus from moving and/or making progression of the stylus more difficult along that side of the ridge 1706. In this example, a first point feature 1702a, a second point feature 1702b, and a third point feature are holes or dimples.

FIG. 18 is a diagram illustrating a patient-specific orbital implant 1800 according to aspects of this disclosure. The implant 1800 includes several characteristics that are similar to those shown in FIGs. 7-17 that may not be discussed further for the purposes of brevity. Here, a first point feature 1802a is a depression at an apex of an elevated ridge 1806. The elevated ridge 1806 includes a side that makes a smooth transition 1808 from the top of the elevated ridge 1806 to the surface of the implant 1800. The smooth transition 1808 allows the stylus to be guided back to a correct position without having to lift the stylus from the surface of the implant 1800 if the stylus is guided too far away from the point features.

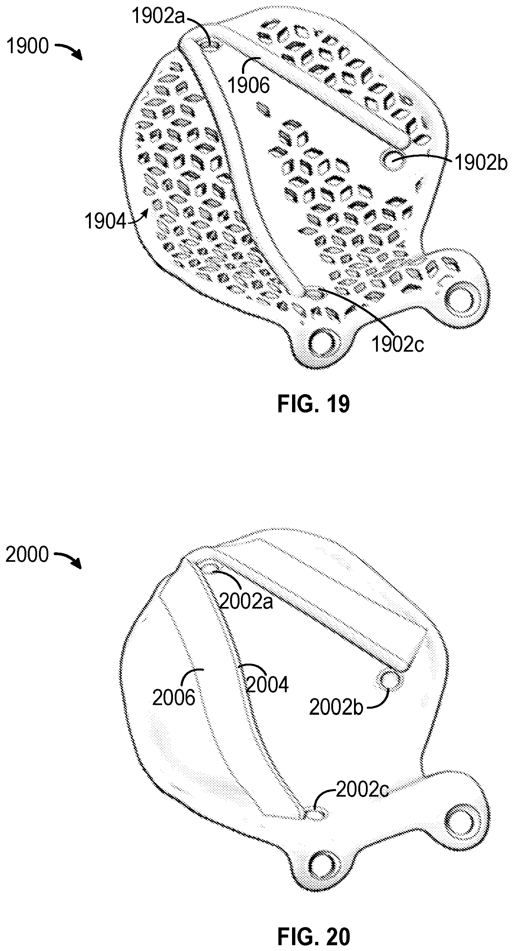

FIG. 19 is a diagram illustrating a patient-specific orbital implant 1900 according to aspects of this disclosure. The implant 1900 includes several characteristics that are similar to those shown in FIGs. 7-18 that may not be discussed further for the purposes of brevity. Here, the implant includes a plurality of perforations 1904, and an elevated ridge 1906 connecting three point features 1902.

FIG. 20 is a diagram illustrating a patient-specific orbital implant 2000 according to aspects of this disclosure. The implant 2000 includes several characteristics that are similar to those shown in FIGs. 7-19 that may not be discussed further for the purposes of brevity. Here, the implant 2000 includes an elevated ridge 2004 with a smooth transition 2006 connecting three point features 2002 characterized by dimples.

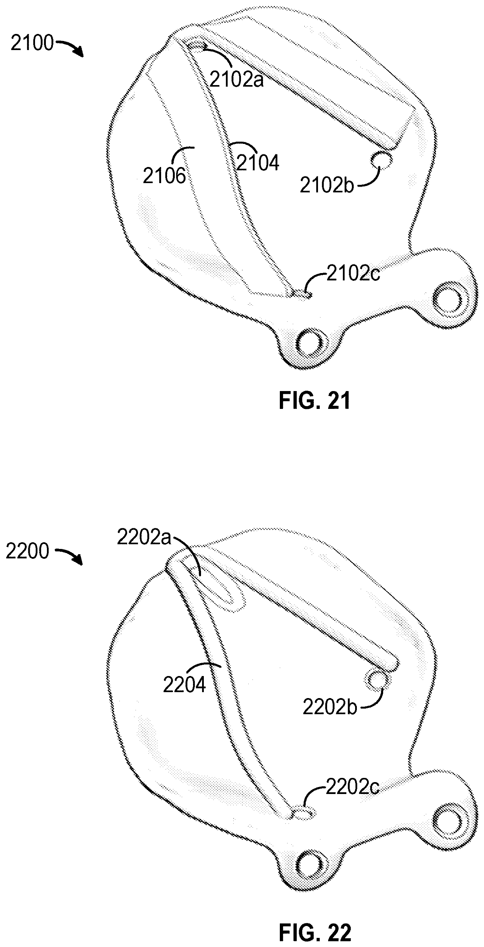

FIG. 21 is a diagram illustrating a patient-specific orbital implant 2100 according to aspects of this disclosure. The implant 2100 includes several characteristics that are similar to those shown in FIGs. 7-20 that may not be discussed further for the purposes of brevity. Here, the implant 2100 includes an elevated ridge 2104 with a smooth transition 2106 connecting three point features 2102 characterized by holes.

FIG. 22 is a diagram illustrating a patient-specific orbital implant 2200 according to aspects of this disclosure. The implant 2200 includes several characteristics that are similar to those shown in FIGs. 7-21 that may not be discussed further for the purposes of brevity. Here, the implant 2200 includes an elevated ridge 2204 connecting three point features 2202. The first point feature 2202a is a depression, while the second point feature 2202b and the third point feature 2202c are dimples.

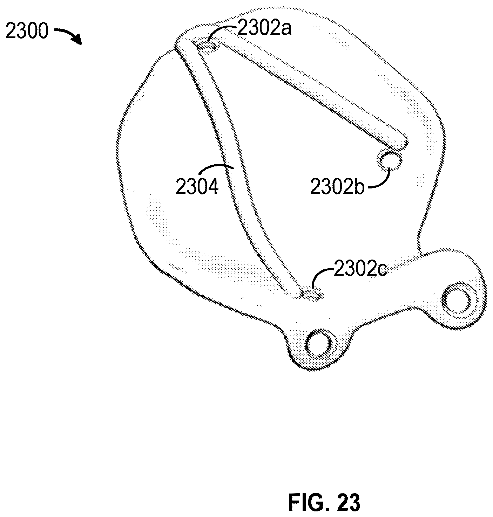

FIG. 23 is a diagram illustrating a patient-specific orbital implant 2300 according to aspects of this disclosure. The implant 2300 includes several characteristics that are similar to those shown in FIGs. 7-22 that may not be discussed further for the purposes of brevity. Here, the implant 2300 includes an elevated ridge 2304 connecting three point features 2302, wherein each point feature is a hole.

FIG. 24 is a diagram illustrating six different example forms of elevated ridges that that may be used in an optical implant. A first elevated ridge 2400a is a "top surface" ridge, meaning the elevated ridge runs along the edge of the implant (e.g., see the elevated ridge 708/808 of FIGs. 7 and 8). A second elevated ridge 2400b is shaped like a wave, having a concave portion of the ridge more able to accept the tip of a stylus. A third elevated ridge 2400c is an elevated ridge that is formed a distance from the edge of the implant (e.g., see the elevated ridge of FIGs. 10-13 and 15-23). A fourth elevated ridge 2400d is shaped like a wave, having a concave portion of the ridge more able to accept the tip of a stylus, and is formed a distance away from the edge of the implant. A fifth elevated ridge 2400e is a plateau type ridge, as discussed above in FIG. 14. And a sixth elevated ridge 2400f is an elevated ridge with a smooth transition from the top of the ridge to the surface of the implant (e.g., see the elevated ridge having a smooth transition from the ridge to the eyeball-facing surface of the implant in FIGs. 18, 20, and 21).

In certain aspects, elevated ridges may be replaced by gutters (e.g., grooves or indentions). Accordingly, an implant according to aspects herein may have non-planar guiding features, which refer to elevated ridges and/or gutters. However, elevated ridges offer the benefit that they do not locally weaken the implant. In other words, they do not reduce the mechanical strength of the implant, or the implant's resistance to deformation. In addition, as described above, in distal areas of the implant, where guidance of the stylus is most needed, the stylus may be angled almost tangentially to the eyeball-facing surface of the implant, which may increase the risk of the tip of the stylus leaving the gutter or may altogether prevent the tip of the stylus from staying in and following a gutter. For that reason, elevated ridges may be preferred.

It is also possible to combine two elevated ridges spaced with a distance between them to establish a channel for guiding the tip of the stylus. However, as with gutters, in distal areas of the implant, the angulation of the stylus with respect to the implant surface may increase the risk of the tip of the stylus leaving the channel or may altogether prevent the tip of the stylus from staying in and following the channel. For that reason, it may be preferred to provide the implant with an elevated ridge or a series of elevated ridges on only one side of a stylus "path" - e.g., the trajectory or range of trajectories that the tip may be expected to follow on its way to a point feature, or the trajectory establishing a path feature - such as the distal side.

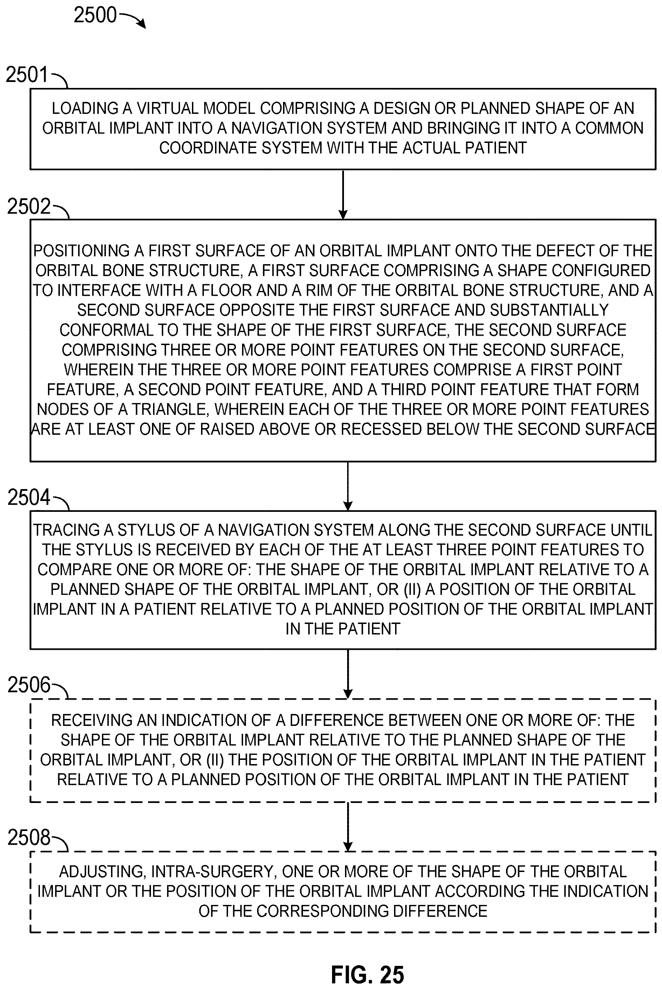

FIG. 25 is a flow chart illustrating operations 2500 for using an orbital implant in accordance with aspects disclosed herein. The operations 2500 may be implemented as software components that are executed and run on one or more processors (e.g., electronic processing device 2606 of FIG. 26) in conjunction with one or more medical instruments (e.g., stylus 2608 of FIG. 26).

At a first block 2501, the operations 2500 include loading a virtual model comprising a design or planned shape of an orbital implant into a navigation system and bringing it into a common coordinate system with the actual patient. For example, a virtual 3D model of a portion of the patient's anatomy may be loaded, wherein the virtual model of the implant and the virtual model of the anatomy are such that the implant is in its planned position with respect to the anatomy of the patient. The actual patient and the virtual model of the anatomy may then be registered (e.g., brought into a common coordinate system). For example, a stylus of the navigation system may be used to indicate anatomical landmarks on the patient anatomy. The locations of these anatomical landmarks may then be registered with the locations of corresponding anatomical landmarks in the virtual model of the anatomy, while maintaining the relative position of the virtual model of the implant with respect to the virtual model of the anatomy.

At a second block 2502, the operations 2500 include positioning a first surface of an orbital implant onto the defect of the orbital bone structure, a first surface comprising a shape configured to interface with a floor and/or a rim of the orbital bone structure, and a second surface opposite the first surface and substantially conformal to the shape of the first surface, the second surface comprising three or more point features on the second surface, wherein the three or more point features comprise a first point feature, a second point feature, and a third point feature that form nodes of a triangle, wherein each of the three or more point features are at least one of raised above or recessed below the second surface.

For example, as discussed with regards to FIGs. 5A-5D, the shape of the first surface may roughly mimic the shape of a surface of a floor and/or a rim of the orbital bone structure, so as to allow a more or less stable positioning of the implant onto the bone structure. Alternatively, as described above for patient-specific implants, a part or the entirety of the shape of the first surface may be designed to closely follow the shape of the bone structure so as to mate with it.

For example, as illustrated in FIGs. 6 and 7, the orbital implant 600/700 may include a plurality of point features 610/702. Specifically, in FIG. 6, the point features 610 may be recessed to the extent that they are perforations in the implant 600. In FIG. 7, the first point feature 702a is a wedge-shaped depression or recess below the second surface, while the second point feature 702b and third point feature 702c are dimples recessed below the second surface.

At a third block 2504, the operations 2500 include tracing the stylus of the navigation system along the second surface until the stylus is received by each of the three or more point features to compare one or more of: (i) the shape of the orbital implant relative to a planned shape of the orbital implant, or (ii) a position of the orbital implant in a patient relative to a planned position of the orbital implant in the patient. For example, a surgeon may trace the stylus along the second surface (e.g., eyeball-facing surface) of the implant, making contact between the stylus and each of the three or more point features. In this way, the physical act of sweeping, or making contact between the stylus and the point features, provides the navigation system with a position of these features in the navigation system's coordinate system. In some examples, making contact between the stylus and a point feature, particularly a point feature near the distal edge of the implant, may comprise sliding the stylus along the second surface in a distal direction until a tip of the stylus hits an elevated ridge of the implant, and then sliding the tip of the stylus along the elevated ridge until it is received in the point feature.

At a fourth block 2506, the operations 2500 may optionally include receiving an indication of a difference between one or more of: (i) the shape of the orbital implant relative to the planned shape of the orbital implant, or (ii) the position of the orbital implant in the patient relative to a planned position of the orbital implant in the patient. For example, once the position of the point features in the navigation system's coordinate system are determined, the navigation system may compare the position of the point features determined by touching the implant with the stylus, with a position of point features of the virtual 3D model of the planned shape of the implant, which was brought into the navigation system's coordinate system in the first block 2501. The navigation system may then determine any differences, such as differences between the constellation of point features in the actual implant and the constellation of point features in the virtual 3D model of the planned shape of the implant, or - derived from such differences - differences between the shape of the actual implant and the planned shape of the implant, and report the differences to the user. In another example, the navigation system may compare the position of the point features determined by touching the implant with the stylus, with a position of point features of the virtual 3D model of the implant relative to the virtual model of the patient's anatomy (e.g., orbital bone structure). In this way, the navigation system may determine a difference between an actual placement of the physical implant relative to the patient's anatomy, and a planned placement of a model of the implant relative to a model of the patient's anatomy.

At a fifth block 2508, the operations 2500 may optionally include adjusting, intra-surgery, one or more of the shape of the orbital implant or the position of the orbital implant according the indication of the corresponding difference. For example, based on the information provided at the fourth block 2506, the user may adjust the implant to more closely match the planned model of the implant, and/or the position of the implant to more closely match the planned position (e.g., virtual positioning) of the implant.

FIG. 26 is a schematic diagram illustrating a conceptual example of a navigation system 2600 with a stylus 2608. The system 2600 can be used in conjunction with preoperative images from an imaging process (e.g., a computed tomography (CT) scan, 3D and/or 2D fluoroscopy, ultrasonic (US) imaging, magnetic resonance imaging (MRI)), and/or the like to perform an image-guided surgical procedure. The system 2600 includes at least an electronic processing device 2606, a display unit 2602, at least one tracked instrument 2608, such as a stylus, and an infra-red sensor 2610. The system 2600 further includes one or more trackers (e.g. a rigidly attached tracker 2612 attached to the patient 2616 anatomy, and optionally another tracker 2614 attached to the stylus 2608). Instruments 2608 and trackers 2612/2614 may comprise markers, which can be easily detected by the infra-red sensor 2610 and which allow the navigation system to locate the instruments 2608 and trackers 2612/2614 in space.

The electronic processing device 2606 can be, for example, a personal computer, or the like. The electronic processing device 2606 includes at least a processor and a memory (e.g., a non-transitory computer-readable medium). The memory (not shown) can be, for example, a random access memory (RAM), a memory buffer, a hard drive, a read-only memory (ROM), an erasable programmable read-only memory (EPROM), and/or so forth. In some embodiments, the memory of the electronic processing device 2606 stores instructions to cause the processor to execute operations (e.g., the operations 2500 of FIG. 25) and/or functions associated with using a personal computer application, controlling one or more medical instruments such as a stylus, displaying and updating an image on display unit 2602 according to movement of the stylus, and/or the like.

The processor (not shown) of the electronic processing device 2606 can be any suitable processing device configured to run and/or execute a set of instructions or code. For example, the processor can be a general purpose processor, a central processing unit (CPU), an accelerated processing unit (APU), or the like. In some embodiments, the processor of the electronic processing device 2606 can be included in, for example, an application specific integrated circuit (ASIC). The processor can be configured to run and/or execute a set of instructions (e.g., the operations 2500 illustrated in FIG. 25) or code stored in the memory associated with using a personal computer application, a mobile application, an internet web browser, telephonic or cellular communication, and/or the like.

The display unit 2602 is configured to be in electronic communication with the electronic processing device 2606. The display 2602 unit can be any suitable display configured to provide a user interface to the electronic processing device 2606. For example, the display unit 2602 can be a cathode ray tube (CRT) monitor, a liquid crystal display (LCD) monitor, a light emitting diode (LED) monitor, a head-mounted device and/or the like. The display unit 2602 can be configured to provide the user interface for a personal computer application or the like. For example, the display unit 2602 can be configured to graphically represent a medical image of an anatomical structure. In some embodiments, the display unit 2602 can graphically represent the position of one or more medical instruments (e.g., the stylus 2608 and/or any other suitable device) as the medical instrument positioned with respect to a target tissue (e.g., an organ and/or bone structure) of a patient and relative to a preoperative image or model of the target tissue. In some embodiments, the processing device 2606 can be configured to map the movement of the stylus 2608 relative to a preoperative image of the target tissue, and the display unit 2602 can graphically represent a virtual position of the stylus 2608 relative to the image of the target tissue. The processing device 2606 can determine the position of the stylus 2608 with respect to the target tissue.

The electronic processing device 2606 may be configured to be in electronic communication with the stylus 2608 (e.g., via a wireless connection, an Ethernet cable, universal serial bus (USB), SATA cable, eSATA cable, or the like). The stylus 2608 can be any suitable instrument configured to be tracked by a tracking system (e.g., optical tracking or other modality). For example, an infrared emitting diode (IRED) may be used to map the position of the stylus 2608 in physical space onto a virtual coordinate system and preoperative image.

### EXAMPLE ASPECTS

Implementation examples are described in the following numbered clauses. The numbered clauses related to implants and methods involving implants. A person skilled in the art will readily appreciate that they may relate to orbital implants or other types of implants.
1. An implant for correcting a defect of a bone structure, comprising: a first surface comprising a shape configured to interface with a surface of the bone structure; and a second surface opposite the first surface and substantially conformal to the shape of the first surface, the second surface comprising three or more point features on the second surface, wherein the three or more point features comprise a first point feature, a second point feature, and a third point feature that form nodes of a triangle, wherein each of the three or more point features are recessed below the second surface.
2. The implant of aspect 1, wherein at least one of the three or more point features comprises a hole through the implant from the first surface to the second surface.
3. The implant of any of aspects 1 and 2, wherein at least one of the three or more point features comprises a depression configured to receive a tip of a stylus angled close to tangentially to the second surface.
4. The implant of aspect 3, wherein the depression is a wedge-shaped or elongated depression.
5. The implant of any of aspects 1-4, further comprising an elevated ridge on the second surface partially surrounding at least one of the three or more point features.
6. The implant of aspect 5, wherein the elevated ridge surrounds the at least one of the three or more point features on a distal side.
7. The implant of any of aspects 5 or 6, wherein the elevated ridge follows a distal edge of the second surface.
8. The implant of any of aspects 5-7, wherein the elevated ridge comprises at least one of a corner, a hook, a notch or a gap at the at least one of the three or more point features.
9. The implant of aspect 8, wherein the corner, hook, notch or gap is configured to receive a tip of a stylus angled substantially tangential to the second surface.
10. The implant of any of aspects 5-9, wherein the elevated ridge extends from a first point feature of the three or more point features to a second point feature of the three or more point features.
11. The implant of any of aspects 5-10, further comprising another elevated ridge on the second surface, the other elevated ridge extending from the second point feature to a third point feature of the three or more point features.
12. The implant of aspect 11, wherein the elevated ridge and the other elevated ridge form a continuous ridgeline on the second surface.
13. The implant of any of aspects 5-12, wherein the elevated ridge comprises a plurality of protrusions at a side of the elevated ridge.
14. The implant of aspect 13, wherein the side is a distal side of the elevated ridge.
15. The implant of any of aspects 5-14, further comprising a gradual transition at a side of the elevated ridge from a top of the elevated ridge to the second surface.
16. The implant of aspect 15, wherein the side is a distal side of the elevated ridge.
17. The implant of any of aspects 5-16, wherein the elevated ridge comprises a concave side for receiving a tip of a stylus.
18. The implant of aspect 17, wherein the concave side is a proximal side of the elevated ridge.
19. The implant of any of aspects 1-4, further comprising one or more elevated ridges extending along a path between a first point feature of the three or more point features and a second point feature of the three or more point features, wherein each of the one or more elevated ridges are located on a first side of the path and not a second side of the path.
20. The implant of aspect 19, wherein the first side of the path is a distal side of the path.
21. The implant of any of aspects 1-20, further comprising a plurality of perforations through the implant from the first surface to the second surface.
22. The implant of aspect 21, wherein a path between a first point feature of the three or more point features and a second point feature of the three or more point features does not include the plurality of perforations.
23. A method for correcting a defect of a bone structure of a patient, the method comprising: positioning a first surface of an implant onto the defect of the bone structure, the first surface comprising a shape configured to interface with a surface of the bone structure, and a second surface opposite the first surface and substantially conformal to the shape of the first surface, the second surface comprising three or more point features on the second surface, wherein the three or more point features comprise a first point feature, a second point feature, and a third point feature that form nodes of a triangle, wherein each of the three or more point features are at least one of raised above or recessed below the second surface; and tracing a stylus of a navigation system along the second surface until the stylus is received by each of the three or more point features to compare one or more of: (i) the shape of the implant relative to a planned shape of the implant, or (ii) a position of the implant in the patient relative to a planned position of the implant in the patient.
24. The method of aspect 23, further comprising loading a virtual 3D implant model into the navigation system and bringing the virtual 3D implant model and the bone structure into a common coordinate system.
25. The method of any of aspects 23 and 24, further comprising loading a virtual 3D anatomy model into the navigation system, wherein bringing the virtual 3D implant model and the bone structure into a common coordinate system comprises: registering anatomical features of the patient with anatomical features of the virtual 3D anatomy model, and maintaining a relative position of the virtual 3D implant model and the virtual 3D anatomy model.
26. The method of any of aspects 23-25, further comprising receiving an indication of a difference between the one or more of: (i) the shape of the implant relative to the virtual 3D implant model, or (ii) the position of the implant in the patient relative to a position of the virtual 3D implant model with respect to the patient.
27. The method of aspect 26, further comprising adjusting, intra-surgery, one or more of the shape of the implant or the position of the implant according the indication of the difference.
28. The method of any of aspects 23-27, wherein at least one of the three or more point features is configured to receive a tip of the stylus angled close to tangentially to the second surface.
29. The method of any of aspects 23-28 wherein the at least one of the three or more point features comprises a wedge-shaped or elongated depression.
30. The method of any of aspects 23-29 wherein the at least one of the three or more point features is partially surrounded by an elevated ridge.
31. The method of aspect 31, wherein the elevated ridge at least partially surrounds the at least one of the three or more point features on a distal side of the at least one of the three or more point features.
32. The method of any of aspects 30 and 31, wherein the elevated ridge comprises a corner, hook, notch or gap at the at least one of the three or more point features for receiving a tip of the stylus of the navigation system.
33. The method of any of aspects 23-32, wherein the implant comprises an elevated ridge, and wherein tracing the stylus of the navigation system along the second surface until the stylus is received by each of the three point features further comprises: sliding a tip of the stylus along the second surface until it hits the elevated ridge; and sliding the tip of the stylus along the elevated ridge until the tip of the stylus is received in at least one of the three point features.
34. A method for generating an implant for correcting a defect of a bone structure, the method comprising: receiving a design of the implant, the design indicating a shape, size, and position of the implant relative to a build area corresponding to an additive manufacturing device, the design of the implant defining: a first surface comprising a shape configured to interface with a surface of the bone structure; and a second surface opposite the first surface and substantially conformal to the shape of the first surface, the second surface comprising three or more point features on the second surface, wherein the three or more point features comprise a first point feature, a second point feature, and a third point feature that form nodes of a triangle, wherein each of the three or more point features are at least one of raised above or recessed below the second surface; and causing manufacturing of the implant using additive manufacturing.
35. The method of aspect 34, wherein at least one of the three or more point features comprises a hole through the implant from the first surface to the second surface.
36. The method of any of aspects 34 and 35, wherein at least one of the three or more point features comprises a depression configured to receive a tip of a stylus angled close to tangentially to the second surface.
37. The method of aspect 36, wherein the depression is a wedge-shaped or elongated depression.
38. The method of any of aspects 34-37, wherein the design of the implant further defines an elevated ridge on the second surface partially surrounding at least one of the three or more point features.
39. The method of aspect 38, wherein the elevated ridge partially surrounds the at least one of the three or more point features on a distal side.
40. The method of any of aspects 38 and 39, wherein the elevated ridge follows a distal edge of the second surface.
41. The method of any of aspects 38-40, wherein the elevated ridge comprises at least one of a corner, a hook, a notch or a gap at the at least one of the three or more point features.
42. The method of aspect 41, wherein the corner, hook, notch or gap is configured to receive a tip of a stylus angled substantially tangential to the second surface.
43. The method of any of aspects 38-42, wherein the elevated ridge extends from a first point feature of the three or more point features to a second point feature of the three or more point features.
44. The method of any of aspects 38-43, wherein the design of the implant further defines another elevated ridge on the second surface, the other elevated ridge extending from the second point feature to a third point feature of the three or more point features.
45. The method of aspect 44, wherein the elevated ridge and the other elevated ridge form a continuous ridgeline on the second surface.
46. The method of any of aspects 38-45, wherein the design of the implant further defines a plurality of protrusions at a side of the elevated ridge.
47. The method of aspect 46, wherein the side is a distal side of the elevated ridge.
48. The method of any of aspects 38-47, wherein the design of the implant further defines a smooth transition at a side of the elevated ridge from a top of the elevated ridge to the second surface.
49. The method of aspect 48, wherein the side is a distal side of the elevated ridge.
50. The method of any of aspects 34-37, wherein the design of the implant defines one or more elevated ridges on one side of a path between a first point feature of the three or more point features and a second point feature of the three or more point features.
51. The method of aspect 50, wherein the one side of the path is a distal side of the path.
52. The method of any of aspects 34-37, wherein the design of the implant further defines an elevated ridge comprising a concave side for receiving a tip of a stylus.
53. The method of aspect 52, wherein the concave side is a proximal side of the elevated ridge.
54. The method of any of aspects 34-37, wherein the design of the implant further defines a plurality of perforations through the implant from the first surface to the second surface.
55. The method of aspect 54, wherein the design of the implant does not define any of the plurality of perforations along a path between a first point feature of the three or more point features and a second point feature of the three or more point features.
56. The method of any of aspects 34-37, further comprising: receiving a digital file comprising the design of the implant; and loading the digital file onto a navigation system.
57. The method of aspect 56, wherein the digital file further comprises data indicative of a position of each of the three or more point features.
58. The method of aspect 57, wherein the data indicative of a position is data indicative of a planned position of the implant with respect to a patient's anatomy.
59. The method of aspect 58, wherein the data indicative of the planned position of the implant comprises a virtual 3D representation of at least part of the patient's anatomy and data indicative of a position of each of three or more point features of the implant with respect to the patient's anatomy.

### ADDITIONAL CONSIDERATIONS

Various embodiments disclosed herein provide for the use of a computer control system. A skilled artisan will readily appreciate that these embodiments may be implemented using numerous different types of computing devices, including both general purpose and/or special purpose computing system environments or configurations. Examples of well-known computing systems, environments, and/or configurations that may be suitable for use in connection with the embodiments set forth above may include, but are not limited to, personal computers, server computers, hand-held or laptop devices, multiprocessor systems, microprocessor-based systems, programmable consumer electronics, network PCs, minicomputers, mainframe computers, distributed computing environments (e.g., networks, cloud computing systems, etc.) that include any of the above systems or devices, and the like. These devices may include stored instructions, which, when executed by a microprocessor in the computing device, cause the computer device to perform specified actions to carry out the instructions. As used herein, instructions refer to computer-implemented steps for processing information in the system. Instructions can be implemented in software, firmware or hardware and include any type of programmed step undertaken by components of the system.

A microprocessor may be any conventional general purpose single- or multi-chip microprocessor such as a Pentium^{®} processor, a Pentium^{®} Pro processor, a 8051 processor, a microprocessor without interlocked pipelined stages (MIPSO) processor, a Power PC^{®} processor, or an Alpha^{®} processor. In addition, the microprocessor may be any conventional special purpose microprocessor such as a digital signal processor or a graphics processor. The microprocessor typically has conventional address lines, conventional data lines, and one or more conventional control lines.

Aspects and embodiments disclosed herein may be implemented as a method, apparatus or article of manufacture using standard programming or engineering techniques to produce software, firmware, hardware, or any combination thereof. The term "article of manufacture" as used herein refers to code or logic implemented in hardware or non-transitory computer readable media such as optical storage devices, and volatile or non-volatile memory devices or transitory computer readable media such as signals, carrier waves, etc. Such hardware may include, but is not limited to, field programmable gate arrays (FPGAs), application-specific integrated circuits (ASICs), complex programmable logic devices (CPLDs), programmable logic arrays (PLAs), microprocessors, or other similar processing devices.

## Claims

1. A method for providing a bending mold for an off-the-shelf implant, the method comprising:
obtaining information about a bony anatomy of a patient from one or more medical images;
developing a virtual three-dimensional model of the bony anatomy based on the information about the bony anatomy;
selecting an off-the-shelf implant;
simulating a bending of the off-the-shelf implant using a three-dimensional model of the selected off-the-shelf implant and the virtual three-dimensional model of the bony anatomy to obtain a virtually bent implant having a first, bone-facing, surface and a second surface opposite the first surface;
using the virtually bent implant, designing a bending mold mating with the first or the second surface; and
based on the design of the bending mold, manufacturing the bending mold using additive manufacturing.

2. The method of claim 1, wherein selecting the off-the-shelf implant comprises selecting an implant type, shape and/or size from a library.

3. The method of claim 1 or 2, further comprising bending the off-the-shelf implant using the bending mold.

4. The method of any one of the preceding claims, wherein the bending mold has a prismatic shape with a flat lower surface and a top surface configured to mate with the first or the second surface.

5. The method of any one of the preceding claims, wherein the bending mold comprises a marking to delineate an outline of the off-the-shelf implant after bending.

6. The method of any one of the preceding claims, wherein the bending mold comprises one or more protrusions configured to be received in one or more notches along an edge of the off-the-shelf implant or perforations of the off-the-shelf implant, such as apertures in the off-the-shelf implant for receiving fixation elements.

7. The method of any one of the preceding claims, wherein the bending mold is manufactured from polyamide.

8. The method of any one of the preceding claims, further comprising choosing a position for the off-the-shelf implant such that fixation points are in locations with good bone stock and delicate anatomical features are not harmed.

9. The method of any one of the preceding claims, wherein the off-the-shelf implant comprises bending locations, such as linear slots or narrowed connections, said bending locations locally reducing the off-the-shelf implant's resistance to deformation and subdividing the off-the-shelf implant into sections.

10. The method of any one of the preceding claims, wherein the off-the-shelf implant comprises a plurality of point features for assessing a location in space of the off-the-shelf implant.

11. The method of claim 10, wherein each of the plurality of point features is configured to receive a stylus of a navigation system.

12. The method of claim 10 or 11, wherein the off-the-shelf implant comprises bending locations subdividing the off-the-shelf implant into sections and wherein each of the plurality of point features is positioned on a section's surface or along a section's edge.

13. The method of any one of claims 10-12, wherein the plurality of point features comprises a hole at an end of a linear slot.

14. The method of any one of the preceding claims, wherein the off-the-shelf implant is an implant for correcting a defect of the bony anatomy, such as an orbital implant.

15. The method of any one of the preceding claims, further comprising providing the bending mold and the off-the-shelf implant.
